# EUROPEAN PATENT APPLICATION

(11) **EP 2 085 904 A2**
(43) Date of publication of application: **05.08.2009**
(21) Application number: 09001094.3
(22) Date of filing: 27.01.2009
(51) Int. Cl.: G06F 19/00

(54) **Medical support control system**

(30) Priority: 29.01.2008 US 21696; 29.01.2008 US 21651; 29.01.2008 US 21799; 26.02.2008 US 37226; 26.02.2008 US 37254
(71) Applicant: Olympus Medical Systems Corporation, Tokyo 151-0072 (JP)
(72) Inventor: Ito, Masaru, Tokyo 151-0072 (JP); Tashiro, Koichi, Tokyo 151-0072 (JP)
(74) Representative: von Hellfeld, Axel

(57) **Abstract**

A medical support control system according to one of aspects of the present invention has a plurality of video interface cards that are used for the medical support control system for converting, when a video signal is input from an external environment, a video signal input from the external environment into a common signal and vice versa, the common signal being different from any video signals input into and output from the plurality of video interface cards and the common signal being used commonly in the medical support control system, a switching control card for selecting, from among the video interface cards, a video interface card as an output destination in accordance with an instruction given from an external environment, and a plurality of video processing cards that are used for the medical support control system for processing the common signal into a video signal appropriate to the selected video interface card on the basis of a video signal expressed by the common signal.

## Description

### Background of the Invention

### Field of the Invention

The present invention relates to a medical support control system for controlling medical devices and non-medical devices used for operations.

### Description of the Related Art

Operating systems using medical controllers or the like for controlling medical devices such as endoscopes or the like used for operations have been proposed. Medical devices to be controlled such as electric knives, insufflation devices, endoscope cameras, light source devices, or the like are connected to the medical controller (also referred to as MC). Also, a display device, a manipulation panel, or the like is connected to the MC. The manipulation panel includes a display unit and a touch sensor, and is used as a central manipulation device by nurses or the like working in an unsterilized area. The display device is used for displaying endoscope images or the like.

There is audio-visual equipment in the operating room such as a room light, a room camera, an interphone device, a liquid crystal display device, or the like (non-medical devices). The audio-visual equipment is controlled independently or by a non-medical controller (also referred to as an NMC) used for the central control.

Japanese Patent Application Publication No. 2006-000536, for example, discloses an operating system, comprising:
a first controller connected to a medical device provided in an operating room;
a second controller connected to a non-medical device provided in the operating room; and
manipulation instruction input means transmitting the content of a manipulation instruction to the first controller when the manipulation instruction to the medical device or the non-medical device is input. The first controller transmits to the second controller a first control signal in accordance with the manipulation instruction of the non-medical device input into the manipulation instruction means. The second controller converts the first control signal into a second control signal used for controlling the non-medical device, and transmits the second control signal to the non-medical device. Thereby, the operating system and a non-medical system work together, and the operating person himself/herself or the like can manipulate the non-medical devices.

### Summary of the Invention

A medical support control system according to one of aspects of the present invention is a medical support control system that can control a medical device, having a plurality of video interface cards that are detachable from the medical support control system and that are used for the medical support control system for converting, when a video signal is input from an external environment, the video signal input from an external environment into a common signal and vice versa, the common signal being different from any video signals input into and output from the plurality of video interface cards and the common signal being used commonly in the medical support control system, a switching control card for selecting, from among the video interface cards, a video interface card as an output destination in accordance with an instruction given from an external environment, and a plurality of video processing cards that are detachable from the medical support control system and that are used for the medical support control system for processing the common signal into a video signal appropriate to the selected video interface card on the basis of a video signal expressed by the common signal.

Preferably, the common signal is a serial signal.

Preferably, all of the respective video interface cards have common connectors.

Preferably, the switching control card determines an output destination for the common signal by detecting a position of a video interface card on the basis of identification information of the video interface card set as an output destination from an external environment, using video interface related information, obtained from the respective video interface cards, containing identification information used for identifying the respective video interface cards and position information specifying a position of the video interface card.

A medical support control system according to one of aspects of the present invention has a plurality of video interface cards that are detachable from the medical support control system that is able to control a medical device and that are used for the medical support control system, an input processing unit for inputting a video signal and an output processing unit for outputting a video signal, a signal conversion unit for converting a common signal into the video signal and vice versa, the common signal being different from any video signals input into and output from the plurality of video interface cards and the common signal being used commonly in the medical support control system, and a common signal input/output unit for inputting and outputting the common signal obtained by the conversion.

A medical support control system according to one aspect of the present invention is a medical support control system that can control a medical device, having a plurality of video interface cards that are detachable from the medical support control system and that are used for the medical support control system for converting, when a video signal is input from an external environment, the video signal input from the external environment into a common signal and vice versa, the common signal being different from any video signals input into and output from the plurality of video interface cards and the common signal being commonly used in the medical support control system, and for detecting a change in an information amount of the input video signal, and a switching control card for determining, when it is determined that the video signal was switched on the basis of the detection result, an output path for the common signal obtained by the conversion on the video signal.

Preferably, the medical support control system further comprises a video processing card for performing image processing, and the switching control card outputs, through a prescribed video interface card, when it is determined that the video signal was switched, the common signal obtained by the conversion on the video signal input via the video processing card.

Preferably, the common signal is a serial signal.

Preferably, the video processing card performs a signal conversion process on a common signal when the video signal was switched.

Preferably, the signal conversion process is SD/HD conversion or HD/SD conversion.

### Brief Description of the Drawings

Fig. 1 shows an entire configuration of a medical device control system according to the present embodiment;
Fig. 2 is a block diagram showing an entire configuration of a medical support control system 100 according to the present embodiment;
Fig. 3 is a side view showing a configuration of the rear panel of an NMC according to the present embodiment;
Fig. 4 shows a configuration of a video interface card;
Fig. 5 shows a configuration of a switching control card;
Fig. 6 shows a configuration of a video processing card;
Fig. 7 shows a flow of signals when a video signal input from the VIC is output to the selected output-destination VIC via the SCC;
Fig. 8 shows a flow of signals when a video signal input from the VIC undergoes image processing in the VIP via the SCC, and is output to the selected output-destination VIC;
Fig. 9 shows a flow of signals when video signals input from a plurality of the VICs undergo image processing in the VPC via the SCC, and are output to the selected output-destination VIC;
Fig. 10 shows a configuration of an audio interface card;
Fig. 11 shows a flow of signals when audio signals input from a plurality of the AICs are output to the selected output-destination AIC via the SCC.
Fig. 12 shows a configuration of a video interface card;
Fig. 13 shows a configuration of a switching control card;
Fig. 14 shows a flow of video signals from an endoscope to the NMC; and
Fig. 15 shows a flow of signals when video signals were switched.
Fig. 16 shows a configuration of a switching control card;
Fig. 17 shows a configuration of a video processing card;
Fig. 18 shows a configuration of a touch panel control card;
Fig. 19 shows a flow of switching signals and video signals from an endoscope to the NMC;
Fig. 20 shows switching of display formats;
Fig. 21 shows a flow of signals when the video signals were switched;
Fig. 22 is a flowchart for a process for controlling the display format in accordance with the switching signals; and
Fig. 23 shows tables for a variation example.
Fig. 24 shows a configuration of a switching control card;
Fig. 25 is a block diagram showing a configuration of a touch panel card;
Fig. 26 shows images created by synthesizing a GUI image and a medical image by using a TPC;
Fig. 27 shows images created by synthesizing the GUI image (including a drawing image) and a medical image by using the TPC;
Fig. 28 shows images created by synthesizing the GUI image and a medical image by using the TPC;
Fig. 29 shows images created by synthesizing the GUI image displayed in the TP and a medical image (including drawing image) displayed in a display device;
Fig. 30 is a block diagram showing a flow of respective image signals when editing is performed; and
Fig. 31 is a flowchart for a process of synthesizing the GUI image and the medical image.
Fig. 32 is a block diagram showing a configuration of an NMC 202 in the present embodiment;
Fig. 33 shows the principle for performing a pre-view of video signals looped back to the NMC 202 according to the present embodiment;
Fig. 34 shows an example of connection between the NMC 202 and a recorder for performing the pre-view of the video signals from the endoscope looped back from the recorder according to the present embodiment;
Fig. 35 shows a communication port setting window 600 for device A according to the present embodiment;
Fig.36 shows an operation window 700 for device A according to the present embodiment;
Fig. 37 is a process flow for a control unit based on the loop-back setting according to the present embodiment;
Fig. 38 is a flowchart for the video signals when looping back is set according to the present embodiment; and
Fig. 39 is a flowchart for the video signals when looping back is not set according to the present embodiment.

### Description of the Preferred Embodiments

Hereinafter, the embodiments of the present invention will be explained in detail, referring to the drawings.

### [First embodiment]

A medical support control system according to the present embodiment includes a medical device control system and a non-medical device control system. The medical device control system includes a plurality of medical devices and a medical controller for controlling these medical devices. The non-medical device control system includes non-medical devices (that may further include medical devices) that are used for operations, and a non-medical controller for controlling these non-medical devices.

An endoscopic operating system will be explained as an example of the medical device control system.

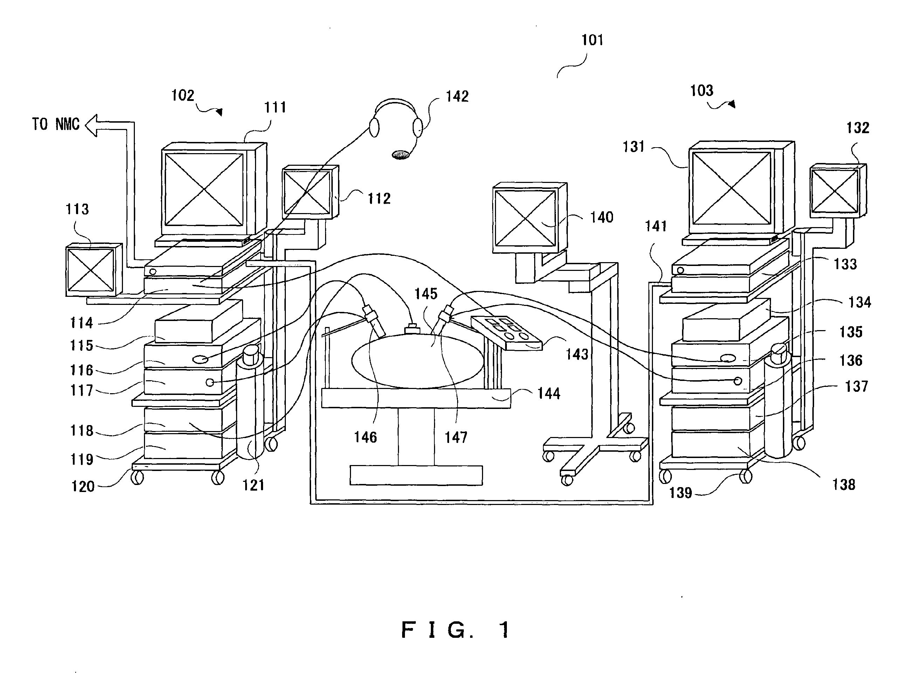

Fig. 1 shows an entire configuration of the medical device control system according to the present embodiment. An endoscopic operating system is shown as a medical device control system 101. In the operating room, a first endoscopic operating system 102 and a second endoscopic operating system 103 beside a bed 144 on which a patient 145 is laid and a wireless remote controller 143 for the operating person are provided.

The endoscopic operating systems 102 and 103 respectively have first and second trolleys 120 and 139 each including a plurality of endoscope peripheral devices used for observation, examination, procedures, recoding, and the like. Also, an endoscope image display panel 140 is arranged on a movable stand.

On the first trolley 120, an endoscope image display panel 111, a central display panel 112, a central manipulation panel device 113, a medical controller (MC) 114, a recorder 115, a video processor 116, an endoscope light source device 117, an insufflation unit 118, and an electrical surgical device 119 are arranged.

The central manipulation panel device 113 is arranged in a unsterilized area to be used by nurses or the like in order to manipulate the respective medical devices in a centralized manner. This central manipulation panel device 113 may include a pointing device such as a mouse, a touch panel, or the like (not shown). By using the central manipulation panel device 113, the medical devices can be managed, controlled, and manipulated in a centralized manner.

The respective medical devices are connected to the MC 114 via communication cables (not shown) such as serial interface cables or the like, and can have communications with one another.

Also, a headset-type microphone 142 can be connected to the MC 114. The MC 114 can recognize voices input through the headset-type microphone 142, and can control the respective devices in accordance with the voices of the operating person.

The endoscope light source device 117 is connected to a first endoscope 146 through a light-guide cable used for transmitting the illumination light. The illumination light emitted from the endoscope light source device 117 is provided to the light guide of the first endoscope 146 and illuminates the affected areas or the like in the abdomen of the patient 145 into which the insertion unit of the first endoscope 146 has been inserted.

The optical image data obtained through the camera head of the first endoscope 146 is transmitted to a video processor 116 through a camera cable. The optical image data undergoes signal processing in a signal processing circuit in the video processor 116, and the video signals are created.

The insufflation unit 118 provides CO₂ gas to the abdomen of the patient 145 through a tube. The CO₂ gas is obtained from a gas tank 121.

On the second trolley 139, an endoscope image display panel 131, a central display panel 132, a expansion unit 133, a recorder 134, a video processor 135, an endoscope light source device 136, and other medical devices 137 and 138 (such as an ultrasonic processing device, a lithotripsy device, a pump, a shaver, and the like) are arranged. These respective devices are connected to the expansion unit 133 through cables (not shown) , and can communicate with one another. The MC 114 and the expansion unit 133 are connected to each other through the expansion cable 141.

The endoscope light source device 136 is connected to a second endoscope 147 through the light-guide cable for transmitting the illumination light. The illumination light emitted from the endoscope light source device 136 is provided to the light guide of the second endoscope 147, and illuminates the affected areas or the like in the abdomen of the patient 145 into which the insertion unit of the second endoscope 147 has been inserted.

The optical image data obtained through the camera head of the second endoscope 147 is transmitted to a video processor 135 through a camera cable. The optical image data undergoes signal processing in a signal processing circuit in the video processor 135, and the video signals are created. Then, the video signals are output to the endoscope image display panel 131, and endoscope images of the affected areas or the like are displayed on the endoscope image display panel 131.

Further, the MC 114 can be controlled by the operating person manipulating the devices in the unsterilized area. Also, the first and second trolleys 120 and 139 can include other devices such as printers, ultrasonic observation devices, or the like.

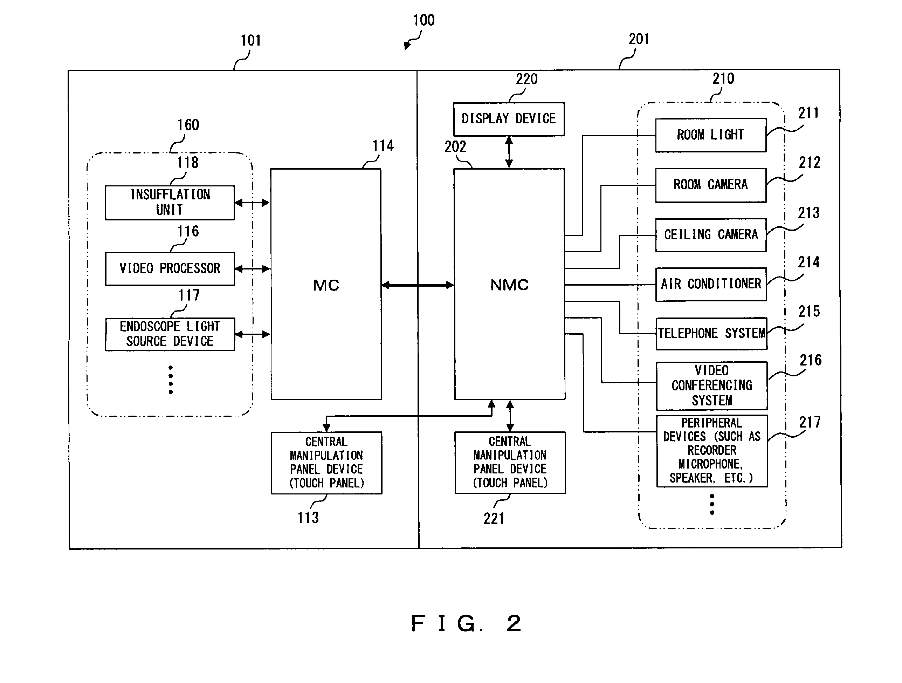

Fig. 2 is a block diagram showing an entire configuration of a medical support control system 100 according to the present embodiment. As described above, the medical support control system 100 includes the medical device control system 101 and a non-medical device control system 201. A detailed configuration of the medical device control system 101 is as shown in Fig. 1. However, in Fig. 2, the medical device control system 101 is shown in a simplified manner for simplicity of explanation.

In Fig. 2, a medical device group 160 is a group of medical devices that are directly connected to the medical controller 114 or are indirectly connected to the MC 114 via the expansion unit 133. Examples of the devices included in the medical device group 160 are the insufflation unit 118, the video processor 116, the endoscope light source device 117, the electrical surgical device 119, and the like.

The central manipulation panel device 113 has a touch panel, and in accordance with the information input into the touch panel, the devices connected to the MC 114 or a non-medical device controller (NMC) 202 that will be described later can be manipulated.

The non-medical control system 201 includes the NMC 202 connected to the MC 114 through a communication cable or the like, and a non-medical device group 210. In this configuration, the NMC 202 can transmit and receive, through an image cable, the video signals to and from the medical device group 160 connected to the MC 114.

The NMC 202 controls the non-medical devices (including the audio-visual devices) connected thereto. As shown in Fig. 2, the non-medical device group 210 connected to the NMC 202 according to the present embodiment consists of a room light 211, a room camera 212, a ceiling camera 213, an air conditioner 214, a telephone system 215, a conference system 216 to be used for individuals in remote places (referred to as a video conference system hereinafter), and other peripheral devices 217. Further, a display device 220 and a central manipulation panel device 221 are connected to the NMC 202.

Also, the non-medical device group 210 includes equipment such as light devices provided in the operating room in addition to the AV devices used for recording and reproducing image data.

The display device 220 is a plasma display panel (PDP) or a liquid crystal display (LCD) device, and displays images of the predetermined device or images of the devices selected by nurses or the like through the central manipulation panel device 221. The room light 211 is a device that illuminates the operating room. The room camera 212 is used for shooting images of the situations in the operating room. The ceiling camera 213 is a camera suspended from the ceiling whose positions can be changed. The conference system 216 is a system that displays images and transmits voices of nurses or the like in the medical office or the nurse stations, and enables conversations with them. The peripheral devices 217 are, for example, a printer, a CD player, a DVD recorder, and the like. The central manipulation panel device 221 has a touch panel that is the same as that included in the central manipulation panel device 113, and controls the respective AV devices connected to the NMC 202. The central manipulation panel devices 113 and 221 are referred to as TPs hereinafter.

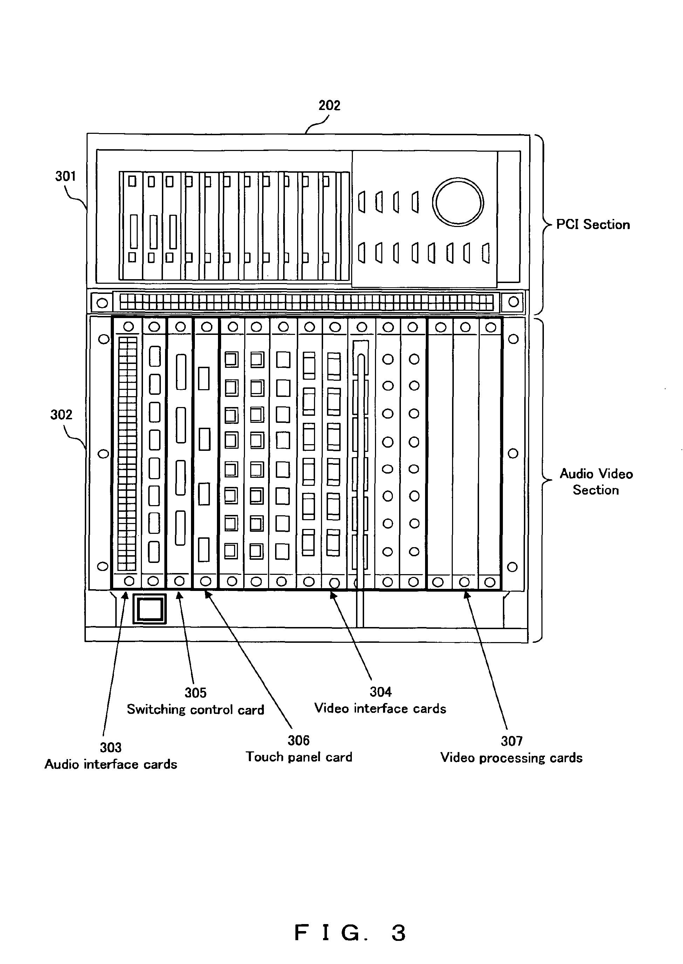

Fig. 3 is a side view showing a configuration of the rear panel of the NMC 202 according to the present embodiment.

The NMC 202 includes a PCI section 301 and an audio/video section 302.

The PCI section communicates with devices connected to the external environment, and has cards having relay devices and the functions of the RS232C, the digital I/O, the ether net, and the modem in order to control devices in the non-medical device group 210 that are connected to other cards that will be described later.

The audio/video section 302 includes audio interface cards 303 (AIC), video interface cards 304 (VIC) , a switching control card 305 (SCC) , a touch panel card 306 (TPC), and video processing cards 307 (VPC). Additionally, the respective cards included in the audio/video section 302 of the NMC 202 are detachable.

The AICs 303 are inserted into a plurality of slots for the AICs 303 in order to receive, process (amplify, for example) , and output audio signals input from a device such as an IC or the like that includes a transmitter/receiver existing in the external environment.

Each of the VICs 304 creates, when a video signal is input into it from the external environment, a common signal, said common signal being different from any of the video signals input into and output from the VICs 304 and said common signal being used commonly in the NMC 202.

In this configuration, examples of the video signals include an HD/SD-SDI (High Definition/Standard Definition-Serial Digital Interface) signal, an RGB/YPbPr signal, an S-Video signal, a CVBS (Composite Video Blanking and Sync) signal, a DVI-I (Digital Visual Interface Integrated) signal, an HDMI (High-Definition Multimedia Interface) signal, and the like.

Also, the VICs 304 have a function of converting the common signals into video signals that are appropriate to the output destinations. Also, the VICs 304 can be inserted into a plurality of slots for the VICs 304. Also, the VICs 304 can have a common interface connector 405. Also, the VICs 304 use paths for directly outputting the input video signals without converting the signals when the VICs 304 are turned off.

The SCC 305 selects the VIC 304 as the output destination in accordance with instructions given from the external environment. Also, the SCC 305 obtains VIC-related information including identification information used for identifying the VICs 304 and position information specifying the positions of the corresponding VICs 304. The identification information is obtained from the VICs 304. Then, the SCC 305 detects, on the basis of the VIC-related information, the position of the VIC 304 as the output destination set in accordance with the instruction given from the external environment, selects the VIC 304 as the output destination for the common signal, and determines whether or not this output should be made via the VPC 307.

The SCC 305 is connected to the TP 221 via, for example, the TPC 306, and the manipulator sets, in the SCC 305, which of the VICs 304 is to be selected as the output destination and whether or not the output should be made via the VPC 307.

The VPC 307, in accordance with the video signals expressed by the common signals, processes the input signals into video signals appropriate to the selected VIC 304.

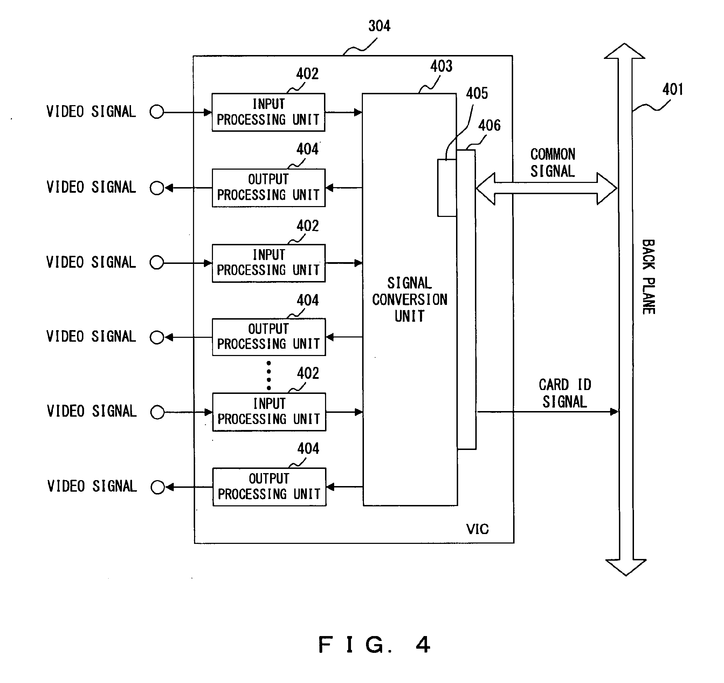

Fig. 4 shows a configuration of the VIC 304.

The VIC 304 is attached to a back plane 401, and includes an input processing unit 402, a signal conversion unit 403, an output processing unit 404, and a connector 405. In this configuration, the back plane 401 includes slots into which the audio interface cards (AIC) 303, the video interface cards (VIC) 304, the switching control card (SCC) 305, a touch panel card (TPC) 306, and the video processing cards (VPC) 307 are inserted. These cards perform communications via the back plane 401.

The VICs 304 transmit and receive, through the back plane 401, the common signals that are obtained by converting the video signals by using the signal conversion unit 403, the common signals input through the cards other than the VICs 304, the identification information for identifying the VICs 304, and the position information for specifying the positions of the slots into which the VICs have been inserted.

The input processing unit 402 receives the video signals output from devices (medical devices and non-medical devices) that are connected to the NMC 202 and are used for outputting video signals, and transfers the received video signals to the signal conversion unit 403.

The signal conversion unit 403 converts the common signal, said common signal being different from any of the video signals input into and output from the VICs 304 and said common signal being used commonly in the NMC 202, into video signals, and vice versa.

In other words, the signal conversion unit 403 converts the video signal input from the input processing unit 402 into the common signals, and outputs the common signals to the back plane 401. Also, the signal conversion unit 403 obtains the common signal input into the VICs 304 via the back plane 401, and converts the obtained signals into video signals appropriate to the selected VIC 304.

Also, the signal conversion unit 403 outputs, via the back plane 401, VIC-related information (a card ID signal) consisting of the identification information used for identifying the VIC 304 and the position information specifying the position of the slot into which the VIC 304 has been inserted.

The output processing unit 404 outputs the video signals obtained by the conversion of the common signal by using the signal conversion unit 403.

The common signal input/output unit 405 inputs and outputs the common signals obtained by the conversion by using the signal conversion unit 403 or the common signals input into the signal conversion unit 403 via the back plane 401.

A connector 406 is attached to the back plane 401. Also, the VICs 304 respectively have input/output terminals that correspond to different video signals from card to card; however, the connector 406 has its shape and pins arranged so that it can be connected to any of the slots for the VICs or the VPC of the back plane 401.

In the conventional techniques, the components of video signals (such as RGB color signals or synchronization signals such as H or V) have been processed independently, which has caused synchronization shifts due to the overtaking of the signals and has caused complexity in the artwork. However, by serializing the common signals that were obtained by the conversion in the VICs 304 as in the present example, artwork (wiring for a PCB) that does not cause the synchronization shifts can be realized easily.

Additionally, in order to be compatible with new image formats that may appear in the future, it is possible to design and manufacture a VIC that corresponds to the new image format and to set a new ID, and thereby the extension to the new format can be realized.

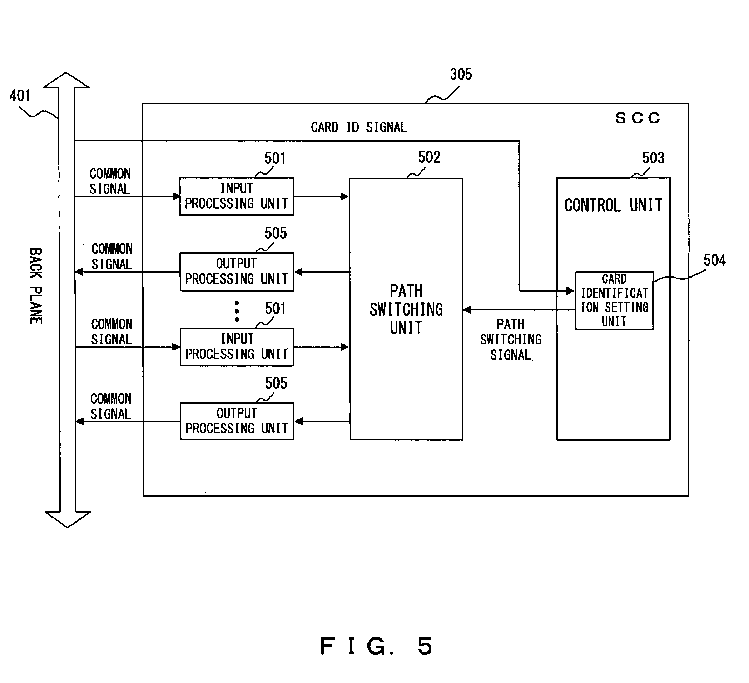

Fig. 5 shows a configuration of the SCC 305.

The SCC 305 is attached to the back plane 401, includes an input processing unit 501, a path switching unit 502, a control unit 503, and an output processing unit 505, and switches the paths for the serialized common signals.

The input processing unit 501 receives the common signals input from the back plane 401 and transfers the received signals to the path switching unit 502.

The path switching unit 502 switches the paths for the common signals. For example, the path switching unit 502 determines, on the basis of the path switching signals output from the control unit 503, the path for the common signal to be output to the output-destination VIC 304. Also, when image processing is to be performed in the VPC 307, the path switching unit 502 determines, on the basis of the path switching signal, the path for the common signal to be output to the output-destination VPC 307. Also, the path switching unit 502 determines the path to the VIC 304 for the common signal that is output from the VPC 307 after the image processing.

The control unit 503 has a card identification setting unit 504, transfers control signals output from an external connection device to the PCI section 301, and obtains control signals input from the PCI section 301 in order to control the respective units in the SCC 305.

The card identification setting unit 504 in the control unit 503 outputs path switching signals that are used for determining the output paths for the output-destination VIC 304 and the VPC 307 on the basis of the identification information and the position information of the VIC-related information (card ID information), and the selection information of the output-destination VIC 304 and the VPC 307 set in accordance with the control signals from the TPs 113 and 221.

In order to perform setting from the external environment, selection information for the output-destination VIC 304 is set in the card identification setting unit 504 from, for example, the TPs 113 and 221 in order to cause the input-destination VIC 304 and the output-destination VIC 304 to correspond to each other. By establishing this correspondence, the position of the output-destination VIC 304 is detected from the VIC-related information in order to determine the output-destination VIC 304 for the common signals.

The output processing unit 505 outputs, to the output-destination VIC 304 set in the above step, the common signals output from the path switching unit 502.

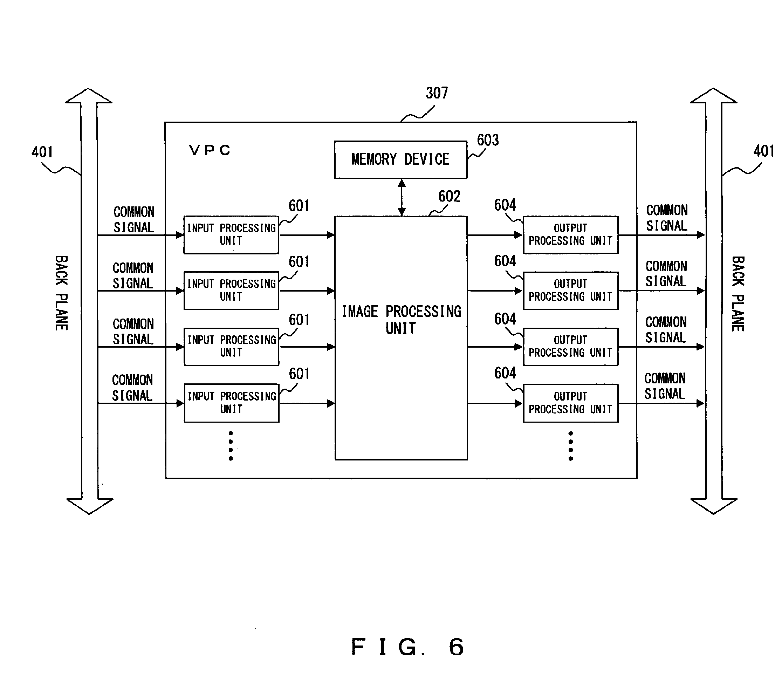

Fig. 6 shows a configuration of a VPC 307.

The VPCs are attached to the back plane 401, and include an input processing unit 601, an image processing unit 602, a memory device 603, and an output processing unit 604.

The input processing unit 601 receives the common signals input from the back plane 401, and transfers the received common signals to the image processing unit 602.

The image processing unit 602, on the basis of the video signals expressed by the common signals, processes the signals into video signals appropriate to the selected VIC 304, and also holds the common signals input from the input processing unit 601 in the memory device 603, and performs image processing on the held common signals in order to output the signals. It is also possible that the common signals undergo the image processing after being converted into the prescribed video signals.

The above image processing includes, for example, the de-interlacing, the rate control, the scaling, the mirror, the rotation, the picture in picture (PIP), the picture out picture (POP), and the like.

The output processing unit 604 transfers, to the SCC 305 via the back plane 401, the common signals that have undergone the image processing performed by the image processing unit 602.

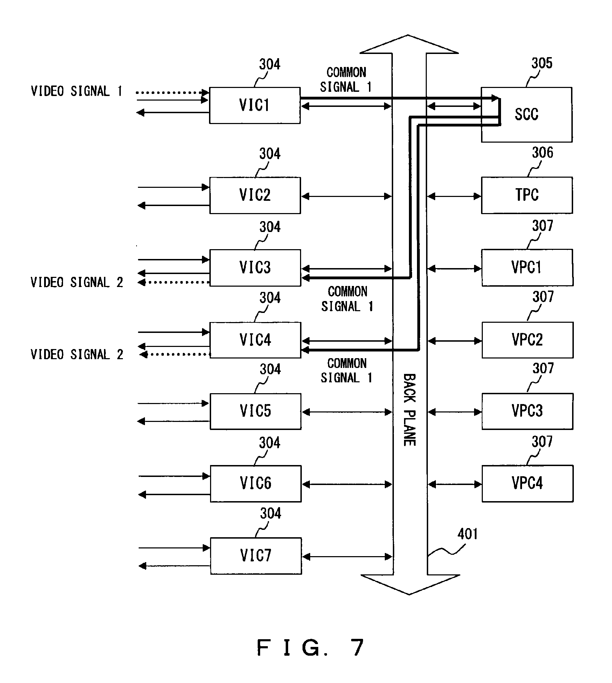

Fig. 7 shows a flow of signals when a video signal input from the VIC 304 is output to the selected output-destination VIC 304 via the SCC 305.

In Fig. 7, an input video signal 1 (represented by a dotted line) is input into a VIC1 304 and is converted into a common signal 1 (represented by a solid line). The converted common signal 1 is input, via the SCC 305, into an output-destination VIC3 304 that is set in advance and the VIC3 304 converts the common signal 1 to the prescribed video signal 2 (dotted line) in order to output the video signal.

It is also possible for the common signal 1 to be divided by the SCC 305 and the common signal 1 to also be input into the output-destination VIC4 304 that is set in advance, and thereafter for the common signal 1 to be converted into a prescribed video signal 2 (represented by a dotted line) in order to be output.

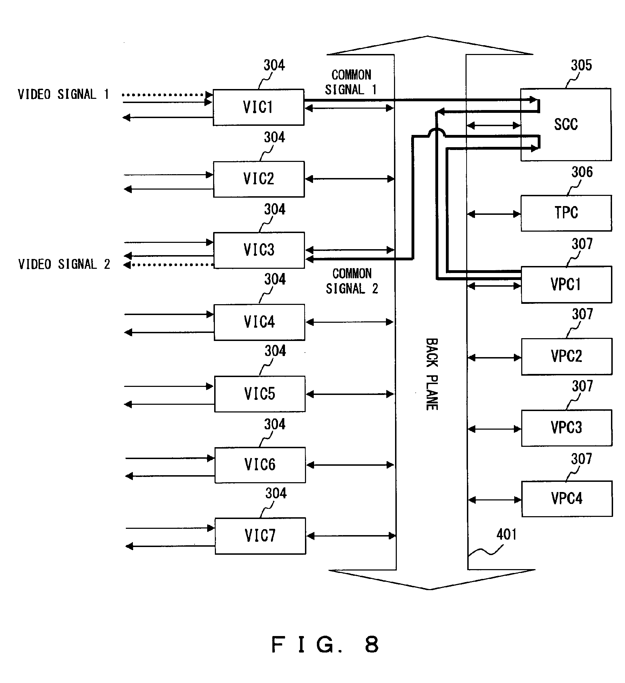

Fig. 8 shows a flow of signals when a video signal input from the VIC 304 undergoes image processing in the VIP 307 via the SCC 305, and is output to the selected output-destination VIC 304.

The video signal 1 (represented by a dotted line) input from an input terminal of the VIC 304 is input into the VIC1 304, and is converted into the common signal 1 (represented by a solid line). The common signal 1 obtained by the conversion is input into the SCC 305, and is input into the output-destination VPC1 307 set by the SCC 305 in advance. In the VPC1 307, signal conversion is performed in which a single video signal image is used or image conversion is performed on the common signal 1 in the VPC1 307. For example, image processing such as the de-interlacing, the rate control, the scaling, the mirror, the rotation, and the like is performed.

The VPC1 307 outputs, to the SCC 305, the common signal 2 obtained by the above signal conversion or the image conversion. The SCC 305 transfers, to the output-destination VIC3 304 set in advance, the common signal 2 obtained by the above signal conversion or the image conversion. The VIC3 304 converts, to the prescribed video signal 2 (represented by a dotted line) , the common signal 2 obtained by the signal conversion, and outputs the signal.

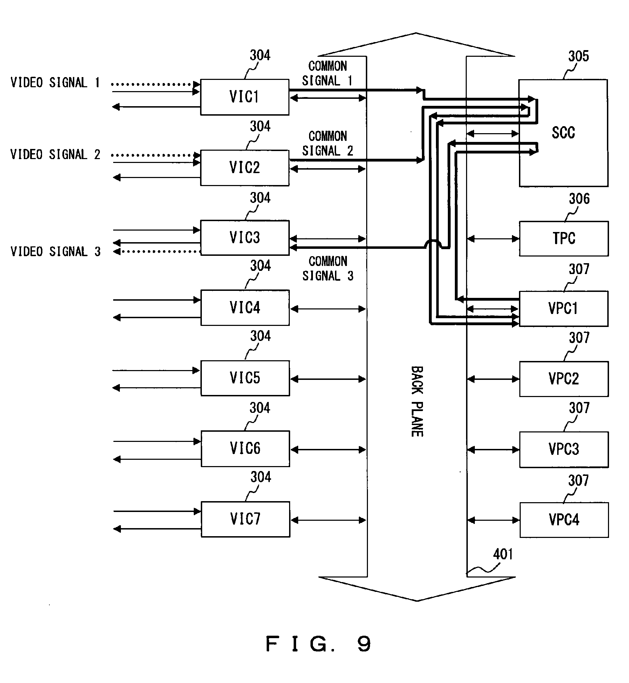

Fig. 9 shows a flow of signals when video signals input from a plurality of the VICs 304 undergo image processing in the VIP 307 via the SCC 305, and are output to the selected output-destination VIC 304.

The video signal 1 (represented by a dotted line) input through an input terminal of the VIC 304 is input into the VIC1 304, and is converted into the common signal 1 (represented by a solid line). Also, the video signal 2 (represented by a dotted line) is input into the VIC2 304, and then is converted into the common signal 2 (solid line) .

The video signals 1 and 2 are respectively converted into the common signals 1 and 2, and these common signals are input into the SCC 305 and are input into the output-destination VPC1 307 that is set in advance by the SCC 305. The VPC1 307 performs on these common signals 1 and 2 an image conversion such as picture in picture (PIP), picture out picture (POP), or the like in which a plurality of video signal images are used.

The VPC1 307 outputs to the SCC 305 a common signal 3 that was obtained by the above image conversion. The SCC 305 outputs the common signal 3 to the output-destination VIC3 304. The VIC3 304 converts the common signal 3 into a prescribed video signal 3 (represented by a dotted line), and outputs the video signal 3.

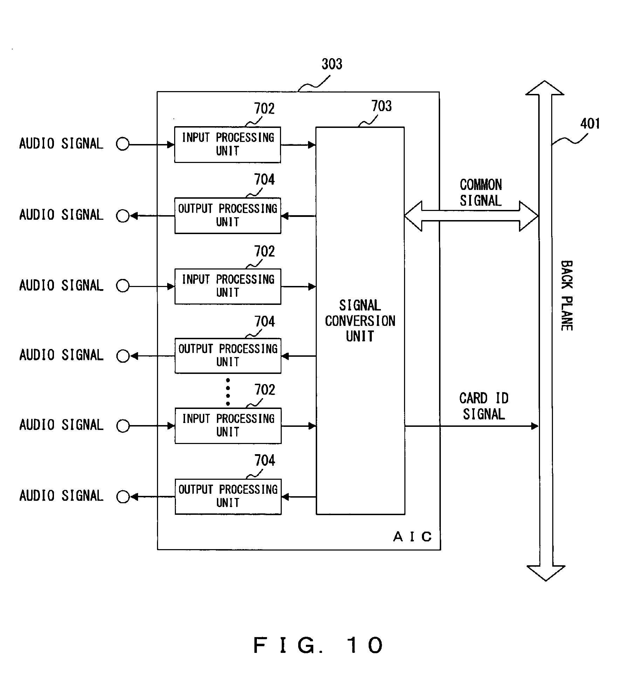

Fig. 10 shows a configuration of the AIC 303.

The AIC 303 is attached to the back plane 401, and includes an input processing unit 702, a signal conversion unit 703, and an output processing unit 704.

The AIC 303 transmits and receives, via the back plane 401, common signals that are audio signals converted by the signal conversion unit 703, common signals input from cards other than the AIC 303, identification information used for identifying the AIC 303, and position information specifying the position of the slot into which the AIC 303 itself is inserted.

The input processing unit 702 receives the audio signals output from the devices (medical device and non-medical devices) connected to the MC 114 and the NMC 202 to be used for outputting the audio signals, and transfers the received signals to the signal conversion unit 703.

The signal conversion unit 703 converts the common signal, said common signal being different from any of the audio signals input into and output from the AICs 303 and said common signal being used commonly in the NMC 202, into audio signals, and vice versa.

In other words, the signal conversion unit 703 converts into the common signals the audio signals input from the input processing unit 702, and outputs the signal obtained by the conversion to the back plane 401. Also, the signal conversion unit 703 obtains the common signals input into the AIC 303 via the back plane 401, and converts the obtained signals into desired audio signals.

Also, the signal conversion unit 703 outputs, via the back plane 401, AIC-related information (a card ID signal) consisting of the identification information used for identifying the AIC 303 and the position information specifying the position of the slot into which the AIC 303 has been inserted.

The output processing unit 704 outputs the audio signals obtained by converting the common signal by using the signal conversion unit 703.

By serializing the common signals that were obtained by the conversion in the AICs 303, artwork (wiring for a PCB) that does not cause synchronization shifts can be realized easily.

Additionally, in order to be compatible with new image formats that may appear in the future, it is possible to design and manufacture an AIC that corresponds to the new image format and to set a new ID, and thereby the extension to the new format can be realized.

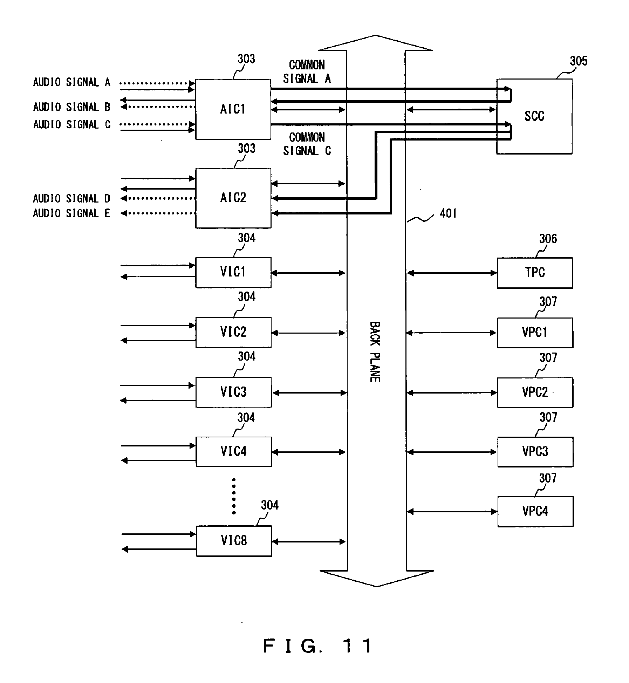

Fig. 11 shows a flow of signals when audio signals input from a plurality of the AICs 303 are output to the selected output-destination AIC 303 via the SCC 305.

In Fig. 11, an input audio signal A (represented by a dotted line) is input into an AIC1 303, and is converted into a common signal A (represented by a solid line). The common signal A obtained by the conversion is input, via the SCC 305, into the output-destination AIC1 303 that was set in advance. Then, the common signal A is converted into a prescribed audio signal B (represented by a dotted line), and the signal obtained by the conversion is output.

Also, an audio signal C (represented by a dotted line) is input into the AIC1 303, and is converted into a common signal C (represented by a solid line). The common signal C obtained by the conversion is input, via the SCC 305, into the output-destination AIC2 303 (different AIC) that is set in advance, is converted into a prescribed audio signal D (represented by a dotted line) or an audio signal E (represented by a dotted line), and is output.

By the above configuration, it is possible to provide a medical support control system for controlling medical devices and non-medical devices.

In the conventional techniques, a different number of inputs and outputs used for images and different image sources have been used depending upon the procedures performed or devices used, and because of this the systems have been large in size and the management of the systems has been complicated.

However, by the above configuration, connection to a plurality of external image conversion devices has become unnecessary and common image cards (such as the VIC and VPC) are employed, which reduces the system size.

Also, when a single device (the NMC 202 shown in Fig. 3) includes the VICs 304, it is possible to change the number of inputs and outputs used for video signals and the types (image sources) of video signals that can be used.

Also, by providing, in a single device, the VPCs 307 each of which can perform a plurality of image processes, it is possible to change the number of types of image processing by changing the VPCs 307 used as occasion.

Also, by using a common back plane and interface connectors that are not dependent on the types of VICs 304 or VPCs 307, it is possible to flexibly use the slots for the VICs 304 and the VPCs 307.

Also, in the conventional techniques, much time has been required to change designs in order to be compatible with new image formats, and external devices have been required, thereby the size of the systems has been large. However, in the present invention, common signals are used, and thereby it is possible to be compatible with new image formats that may appear in the future by designing only VICs that correspond to the new image formats without changing the design greatly.

The scope of the present invention is not limited to the above embodiments, and various alterations and modifications are allowed without departing from the spirit of the present invention.

### [Second embodiment]

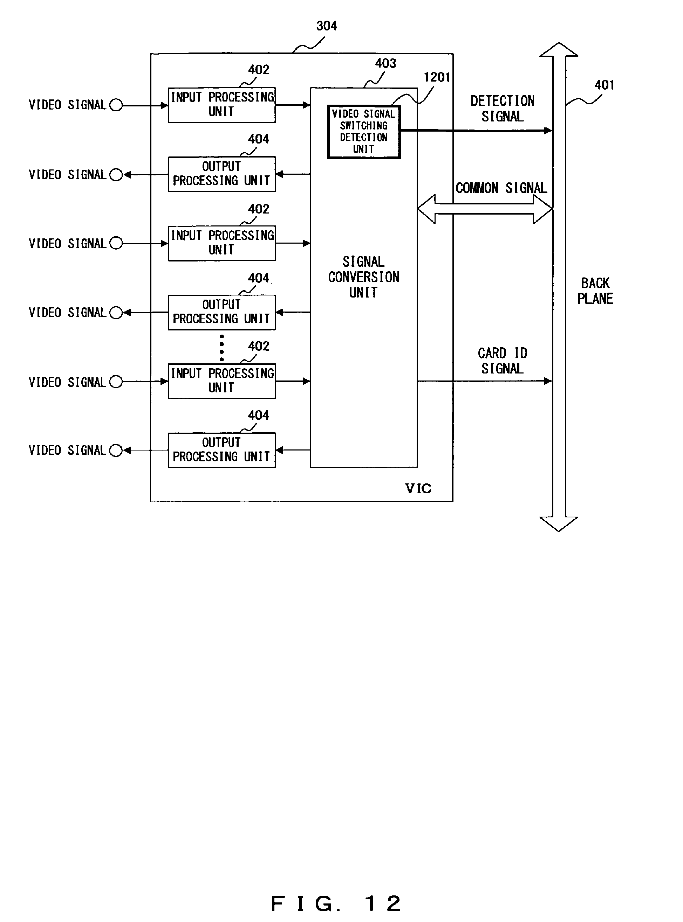

Fig. 12 shows a configuration of the VIC 304 in a second embodiment.

The VIC 304 is attached to a back plane 401, and includes an input processing unit 402, a signal conversion unit 403, an output processing unit 404, and a video signal switching detection unit 1201. In this configuration, the back plane 401 includes slots into which the audio interface cards (AIC) 303, the video interface cards (VIC) 304, the switching control card (SCC) 305, a touch panel card (TPC) 306, and the video processing cards (VPC) 307 are inserted. These cards perform communications via the back plane 401.

The video signal switching detection unit 1201 detects an amount of information of the video signals input from the input processing unit 402. When, for example, HD-SDI signals, i.e., signals that are not SD-SDI signals, are input into the input processing unit 402 which outputs the signals to the SCC 305 after obtaining the SD-SDI signals and converting them into common signals, the video signal switching detection unit 1021 detects the fact that the video signals were switched on the basis of the information amount of the video signals (for example, the resolution, whether it is interlaced/progressive, the frame rate, or the like), and transmits a detection signal to the SCC 305 via the back plane 401.

Additionally, even though the video signal switching detection unit 1201 is provided in the signal conversion unit 403, the video signal switchingdetection unit 1201 can be provided in each of the input processing units 402.

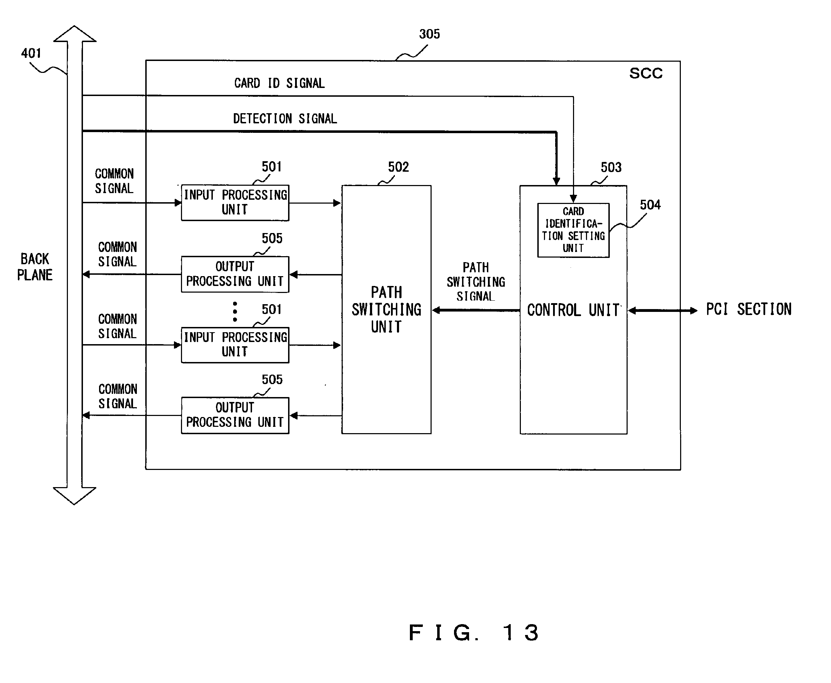

Fig. 13 shows a configuration of the SCC 305.

The control unit 503 includes a card identification setting unit 504 in order to transfer control signals input into the PCI section 301 from the external connection device and to control the respective units in the SCC 305 by obtaining control signals input from the PCI section 301.

Also, when it is determined that the detection signal created in the VIC 304 was obtained and the video signals input from the VIC 304 were switched, an output path for a common signal obtained by converting the video signal is changed. The output path for the common signal is such that the common signal goes from the SCC 305 to the VPC 307 where the signals are converted into the other type of common signal and is output again via the SCC 305 to a prescribed VIC 304. This setting of the path is also performed on the basis of the path switching signal.

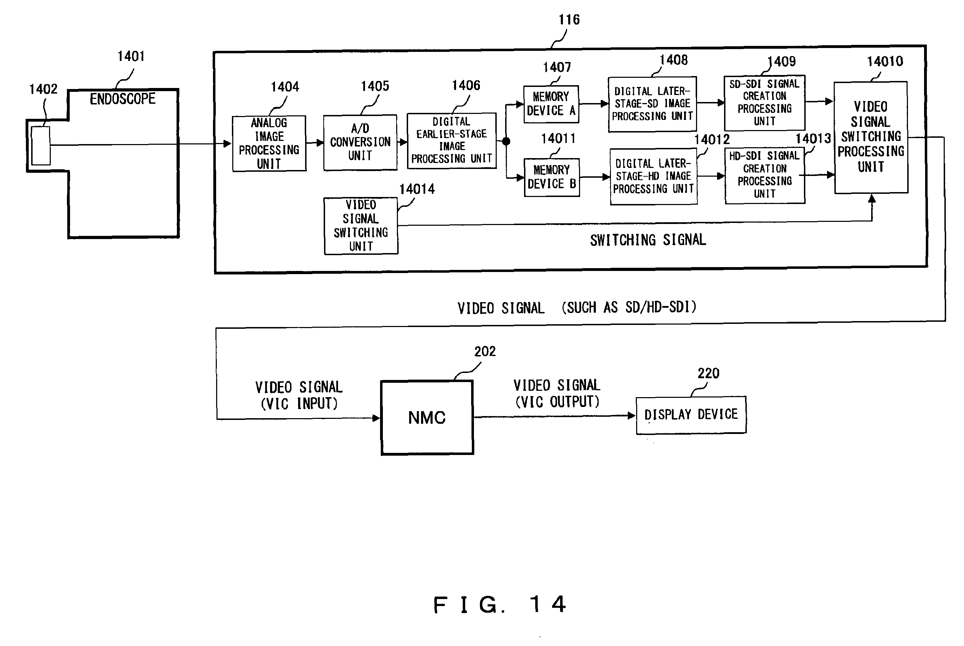

The endoscope system shown in Fig. 14 includes an endoscope 1401, a video processor 116, the MC 114, and the NMC 202.

The endoscope 1401 has various CCDs 1402, and is connected to the endoscope light source device 117 and the video processor 116. The endoscope 1401 performs the endoscopic examination or the like by being inserted into body cavities. The endoscope light source device 117 is detachably connected to the connector of the endoscope 1401 to which illumination light is to be provided.

The video processor 116 detachably connected to the endoscope 1401 can perform signal processing corresponding to various CCDs 1402. Additionally, only one endoscope 1401 is shown in Fig. 14; however, in the actual configuration, a plurality of types of endoscopes whose CCDs 1402 yield different numbers of pixels (resolutions) are connected to the video processor 116.

The CCDs 1402 correspond respectively to two types of video signals, the SDTV signal (standard-definition TV signal) and the HDTV signal (such as a high-definition video signal).

The video processor 116 is a signal processing device for endoscope signals used for performing signal processing for image shooting means included in the endoscope 1401; it receives video signals via the connector that is detachably connected and transfers to the NMC 202 the endoscope images shot by the image shooting means.

The video processor 116 has a function of performing signal processing in which SD signals appropriate to the CCD 1402 included in the endoscope 1401 are created and a function of performing signal processing in which HD signals are created.

Because the endoscope 1401 is connected to the video processor 116, the CCD driver (not shown) provided in the video processor 116 applies CCD drive signals to the CCD 1402. The CCD 1402 outputs, to an analog image processing unit 1404 in the video processor 116, CCD output signals that have undergone photoelectric conversion performed with the CCD drive signals applied.

The output signals from the above CCD 1402 undergo processing such as amplification, correlated double sampling, or the like performed by the analog image processing unit 1404, are input into an A/D conversion unit 1405, and are converted from analog to digital.

This digital signal is input into a digital earlier-stage image processing unit 1406, undergoes processing such as a color division process in which the signal is divided into luminance signals and color signals, and also undergoes matrix processing in which the luminance signals and the color signals are converted into RGB signals, a white balance signal, and the like; thereafter, the divided signals are temporarily stored in two memory devices (a memory device A 1407 and a memory device B 14011). The signal read from these two memory devices undergoes a signal processing corresponding to the SD signals (standard-definition video signals) and the HD signal (high-definition video signal) yielding higher resolution than the SD signal, as will be explained later.

The signal read from the memory device A 1407 is input into a digital later-stage-SD image processing unit 1408, and in this digital later-stage-SD image processing unit 1408, processing such as enlargement processing, enhancement processing, or the like is performed on the basis of SDTV. Thereafter, the output signal from this digitallater-stage-SD image processing unit 1408 is input into an SD-SDI signal creation processing unit 1409 that converts signals into serial video signals. The SD-SDI signal creation processing unit 1409 has a serial digital interface (SDI), and converts the SD signals into serial video signals.

Also, the signal read from the memory device B 14011 is input into a digital later-stage-HD image processing unit 14012. Then, in this digital later-stage-HD image processing unit 14012, processing such as enlargement processing, enhancement processing, or the like is performed on the basis of HDVT.

SD and HD have different aspect ratios, and accordingly the digital later-stage-SD image processing unit 1408 and the digital later-stage-HD image processing unit 14012 correspond to the respective aspect ratios and perform the same processes.

Thereafter, the output signal of this digital later-stage-HD image processing unit 14012 is input into an HD-SDI signal creation processing unit 14013 for converting the signal into serial video signals. The serial output signals of the SD-SDI signal creation processing unit 1409 and the HD-SDI signal creation processing unit 14013 are input into the display device 220 via a video signal switching processing unit 14010 and the NMC 202.

The video signal switching processing unit 14010 outputs the video signals selected on the basis of the switching signal for the video signals that are the SD or HD selection instruction given from, for example, a video signal switching unit 14014.

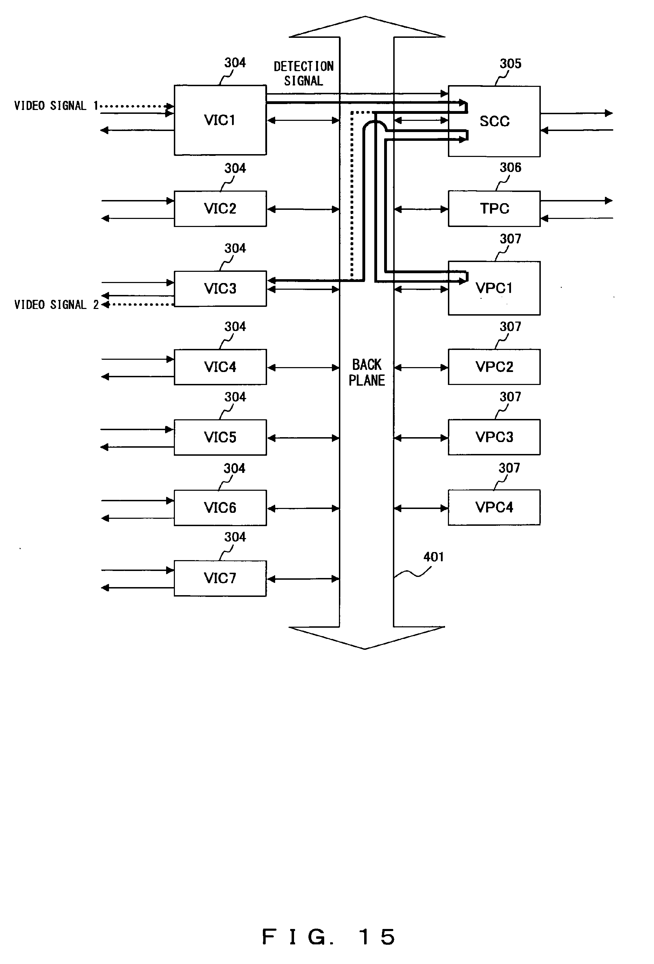

Fig. 15 shows a flow of signals when video signals transferred from the medical device group 160 such as the endoscope system or the like are displayed on the prescribed display device 220. In Fig. 15, explanations are given on the assumption that the video signal input into the VIC 304 is the SD-SDI signal or the HD-SDI signal that was transferred from the endoscope 1401 (explained in Fig. 14).

The video signals input into the VIC 304 are converted into common signals, and are output, via the SCC 305, to the output-destination VIC 304 that is set by the SCC 305. However, when an input device (such as a keyboard, a mouse, or the like) connected to the video processor is used to switch the video signals, the image being output is intermitted.

In order to cope with this phenomenon, an attempt is made so that images being displayed on the display device 220 used as an output device are not intermitted even when the video signals are switched. For example, signals are output so that even when the SD-SDI signals are switched to the HD-SDI signals, images being displayed on the output device corresponding to the SD-SDI signals are not intermitted.

The video signal switching detection unit 405 shown in Fig. 12 detects the fact that the video signals were switched by the VIC 304. Then, the video signal switching detection unit 1201, by using the detection signal, notifies the control unit 503 in the SCC 305 of the fact that the video signals were changed. On the basis of this detection signal, the SCC 305 again sets the path for outputting the signal to the output-destination VIC 304 via the VPC 307.

When, for example, the SD-SDI signals that have been input into the SD/HD-SDI input terminal in the VIC 304 are switched to HD-SDI signals, the HD-SDI signals are converted into common signals, and are transferred to the SCC 305 and the VPC 307 by the VIC 304. In the VPC 307, the common signals (HD-SDI) are converted into the common signals (SD-SDI) (HD/SD conversion).

Inversely when the HD-SDI signals are changed to the SD-SDI signals, the VPC converts the common signals (SD-SDI) into the common signals (HD-SDI) (SD/HD conversion).

Additionally, in the above configuration, the SD/HD conversion has been explained; however, the signal conversion for the RGB/YPbPr, S-Video, CVBS, DVI-I, and HDMI is also performed in the same manner when switching occurs.

In this configuration, the VPC 307 can directly convert common signals, and also can convert the common signals again after converting the common signal into image-like signals. "Image-like signals" means common signals in a state that is necessary for the VPC 307 in order to perform conversion processing. For example, the signal is converted into image signals such as the SD-SDI, RGB/YPbPr, S-Video, CVBS, DVI-I, and HDMI.

In Fig. 15, before the signal switching occurs, a video signal 1 is input into the VIC1 304 and the common signal obtained by converting the video signal 1 passes through the SCC 305, and is converted again into a prescribed video signal 2 in the VIC3 304 and is output (the path represented by the dotted line in Fig. 15).

When the switching has occurred, the video signal 1 input from the VIC1 304 is transferred to the SCC 305 after making the changed video signal 1 the common signal. Thereafter, the common signal is transferred to the VPC1 307 on the basis of the switching path of the SCC 305 that was set again in accordance with the detection signal. When the signal conversion is completed in the VPC1 307, the converted common signal is transferred to the SCC 305, and is transferred to the prescribed VIC3 304; thereafter the video signal 2 is output.

When XGA is fixed in the setting for the output devices such as the display device 220, any signal regardless of whether it is the SD signal or the HD signal is transmitted via the VPC 307.

Also, when the output device has an automatic switching function for video signals, video signals that can be switched by the output device are recorded in advance, and the current path (VIC 304 → SCC 305 → VIC 304) is maintained without setting the path via the VPC 307 even when switching to the recorded video signal.

Further, when a common signal has the same resolution, frame rate, and scanning method, it is possible for the signal to be transmitted via a route other than the VPC 307. For example, when the S-Video signal (NTSC) is changed to the CVBS signal (NTSC), the S-Video signal is changed to the common signal in the VIC 304, is converted into the CVBS signal (NTSC) in the output-destination VIC 304, and is output.

By using the above configuration, a medical support control system that can control medical devices and non-medical devices can be provided.

In the above configuration, even when the format of the input signals is changed, conversion into video signals appropriate to the output devices can be performed on the basis of the result of the automatic detection, and accordingly the above problem can be avoided.

In other words, the conventional problem can be avoided, in which, if a user changes a signal to one that is not compatible with the device when the format of signals of the display devices is limited, the image disappears (for example, the displayed image or the recorded image is blacked out), or the change causes damage to devices.

"Damage to devices" includes the situation in which non-standard video signals are successively input into a device which is included in a display device and receives video signal. For example, a situation in which signals are successively input at a level above the standard.

Also, users can switch signals without being concerned about the input format of the output devices.

The scope of the present invention is not limited to the above embodiments, and various alterations and modifications are allowed without departing from the spirit of the present invention.

### [Third embodiment]

Also, the VICs 304 in the third embodiment have a function of converting the common signals into video signals that are appropriate to the output destinations. Also, the VICs 304 can be inserted into a plurality of slots for the VICs 304. Also, the VICs 304 can have a common interface connector 405. Also, the VICs 304 use paths for directly outputting the input video signals without converting the signals when the VICs 304 are turned off.

The VIC 304 is attached to a back plane 401, and includes an input processing unit 402, a signal conversion unit 403, an output processing unit 404. In this configuration, the back plane 401 includes slots into which the audio interface cards (AIC) 303, the video interface cards (VIC) 304, the switching control card (SCC) 305, a touch panel card (TPC) 306, and the video processing cards (VPC) 307 are inserted. These cards perform communications via the back plane 401.

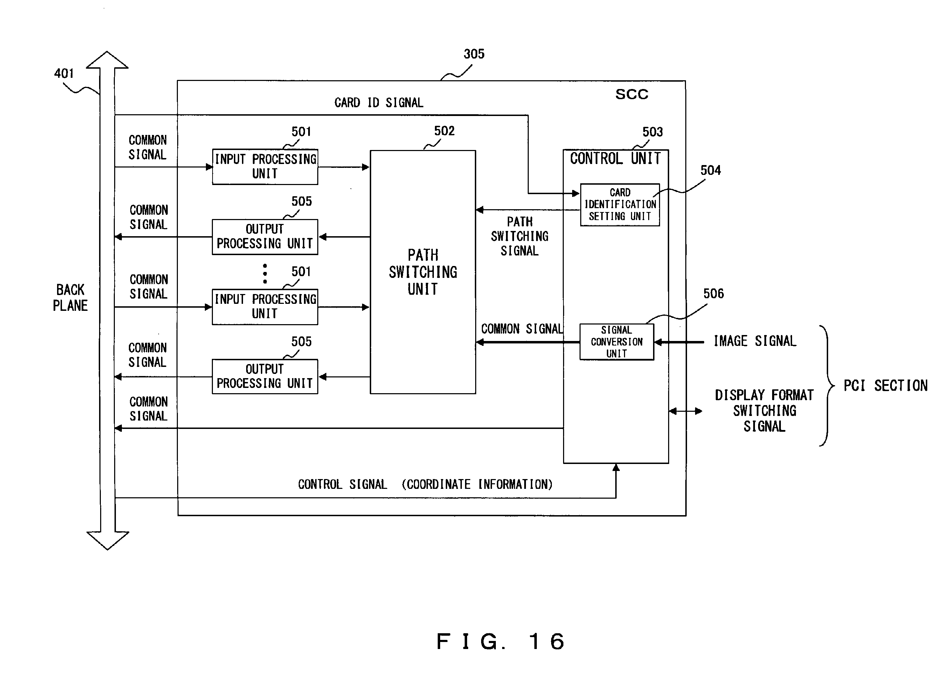

Fig. 16 shows a configuration of the SCC 305 in the third embodiment.

The control unit 503 has a card identification setting unit 504 and a signal conversion unit 506, transfers control signals (including display format switching signals) input into the PCI section 301 from the endoscope, and controls the respective units in the SCC 305 by obtaining the control signals input from the PCI section 301.

Also, the control unit 503 obtains the control signals (including display format switching signals) transferred from the PCI section 301, and transfers, to the back plane 401 via the VPC 307, the display format switching signals used for switching the image processing of the VPC 307.

The signal conversion unit 506 converts into common signals the image signals (such as GUI image signal) transferred from the PCI section 301, and transfers the signals to the path switching unit 502.

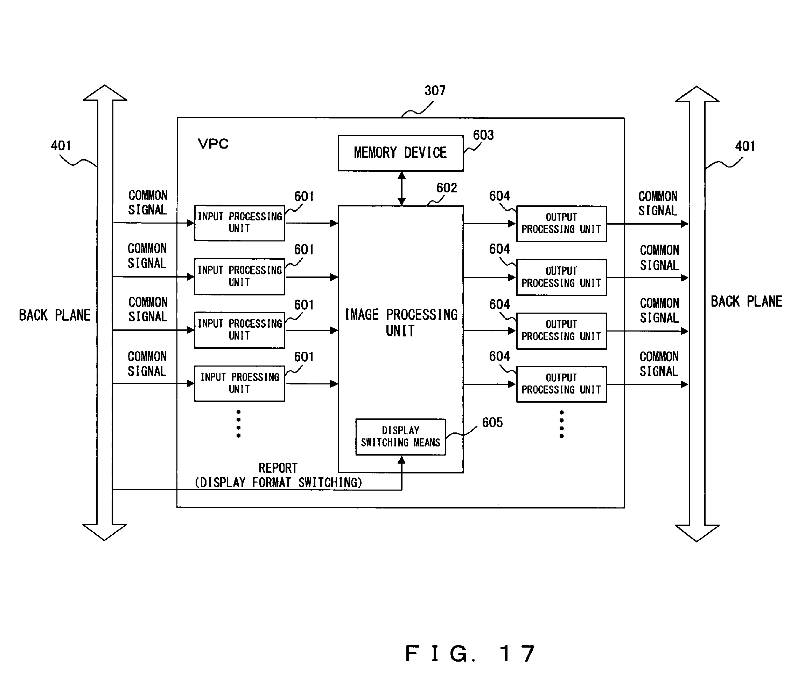

Fig. 17 shows a configuration of a VPC 307 in the third embodiment.

The VPCs are attached to the back plane 401, and include an input processing unit 601, an image processing unit 602, a memory device 603, and an output processing unit 604.

A display switching means 605 causes the display device to sequentially display images in formats according with the display format switching signals transmitted from the manipulation unit in the medical device.

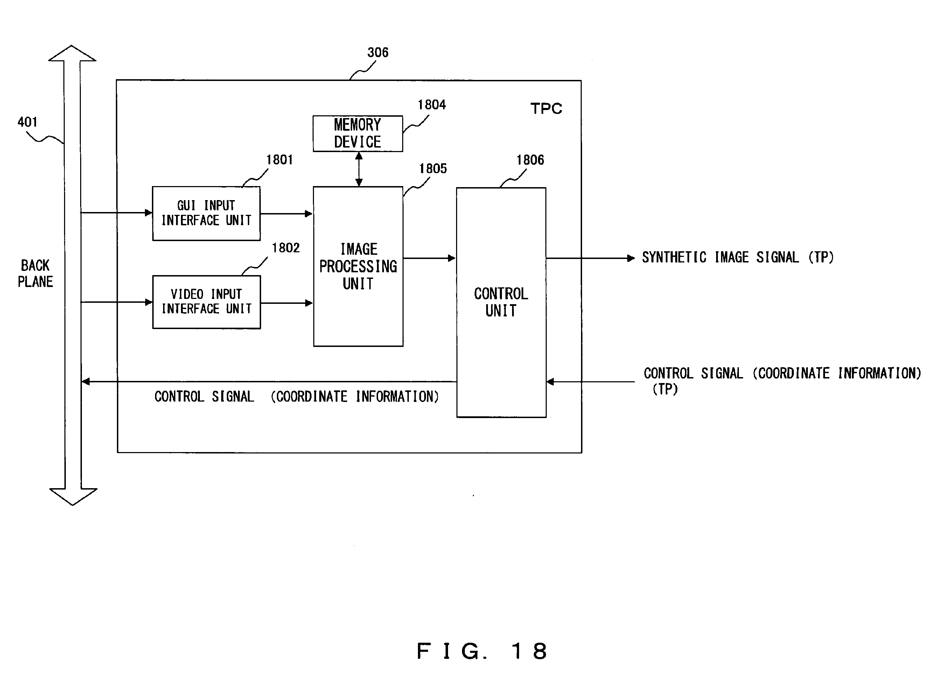

Fig. 18 shows a configuration of the touch panel card 306 in the third embodiment.

The TPC 306 is included in the back plane 401, and has a function of editing images in accordance with the instructions given by the TPs 113 and 221.

The TPC 306 includes a GUI input interface unit 1801, a video input interface unit 1802, a memory device 1804, an image processing unit 1805, and a control unit 1806. The GUI input interface unit 1801 and the video input interface unit 1802 collect images in accordance with the instructions given by the TPs 113 and 221.

The GUI input interface unit 1801 is an interface that obtains display layout information (hereinafter, referred to as a GUI (Graphical User Interface) image) created in the PCI section 301, and transfers the image to the image processing unit 1805 via the SCC 305 and the back plane 401.

The video input interface unit 1802 obtains medical images from the medical device group 160, and outputs the data obtained from the images to the image processing unit 1805.

In this configuration, the medical images are input into the VIC 304 as video signals, are converted into common signals in the VIC 304, and are input into the SCC 305. Thereafter, the medical images that have been converted into the common signals are output to the output-destination VPC 307 on the basis of the setting in the SCC 305, undergo image processing in the VPC 307, and are input into the video input interface unit 1802.

Thememorydevice 1804 holds the GUI images obtained in the GUI input interface unit 1801, the medical images obtained in the video input interface unit 1802, or the synthetic images processed in the image processing unit 1805.

The image processing unit 1805 performs image processing on the respective images obtained in the GUI input interface unit 1801 and the video input interface unit 1802, and transfers the image-processed images to the control unit 1806. In other words, by including the medical images in a prescribed region in the GUI image, the GUI image and the medical image are synthesized.

The control unit 1806 directly outputs the images that have undergone the image processing in the TP 221. Also, the control unit 1806 is a device for controlling the entirety of the TPC 306, and outputs the images that have undergone the image processing in the image processing unit 1805 to the TPs 113 and 221.

Also, control signals (coordinate information) transferred from the TPs 113 and 221 are output to the back plane 401, and are transferred to the SCC 305.

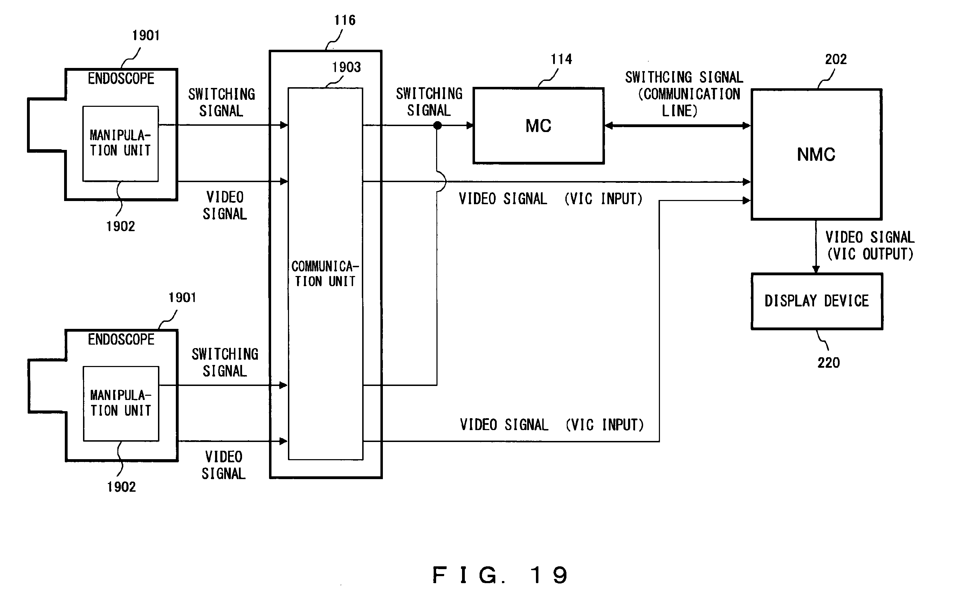

Fig. 19 shows a configuration including the endoscopes 1901 that are used as external control devices arranged in the sterilization area and the medical support control system. The example in Fig. 19 includes the endoscopes 1901, the video processor 116, the MC 114, the NMC 202, and the display device 220.

A plurality of the endoscopes 1901 have switch devices used for switching the display formats of medical images, and manipulation units 1902 for detecting the switching instructions given by the switch devices.

The manipulation unit 1902 outputs switching signals to the video processor 116 when detecting the switching instruction given by, for example, the switch device.

When receiving a switching signal, the communication unit 1903 in the video processor 116 transfers this switching signal to the MC 114.

The MC 114, having received the switching signal, transfers the switching signal to the NMC 202 via, for example, a communication line.

The NMC 202 causes the display device to display images in the switched display formats each time the NMC 202 receives the switching signal. The display format means the results of the image processing on medical images output from the endoscopes 1901.

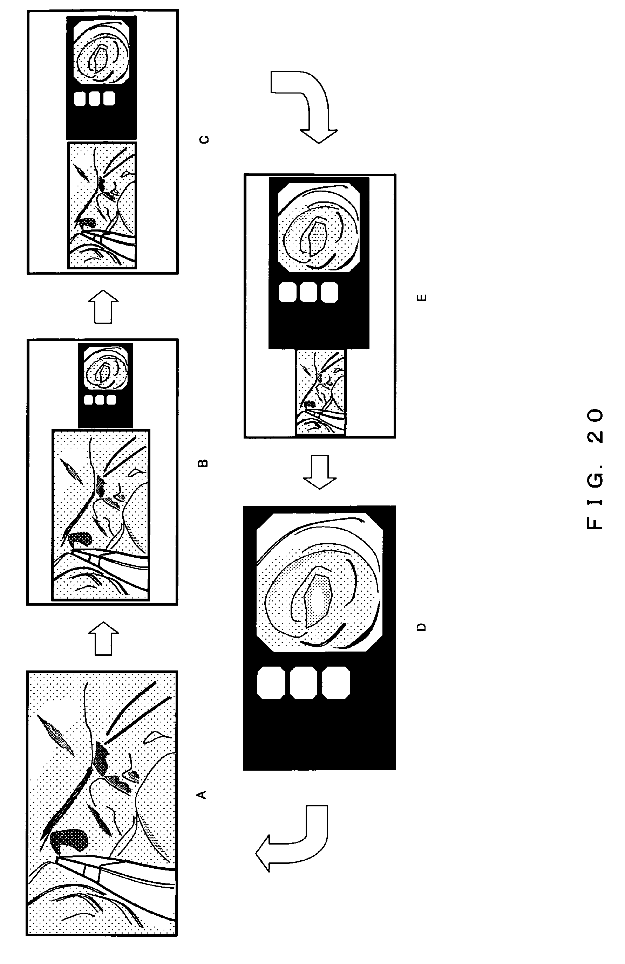

Fig. 20 shows display formats. The images shown in Fig. 20 are medical images that are switched in accordance with the switching instructions given by the endoscopes 1901. In Fig. 20A, a medical image transferred from the endoscope 1901 is displayed at full screen. In Fig. 20B, the medical image displayed in Fig. 20A and a medical image shot by a medical device other than the endoscope 1901 are displayed at different sizes. In Fig. 20C, the two medical images in Fig. 20B are displayed at the same size. In Fig. 20D, a medical image shot by a medical device other than the endoscope 1901 is displayed at full screen. In Fig. 20E, the images are displayed in sizes reversed from those in Fig. 20B.

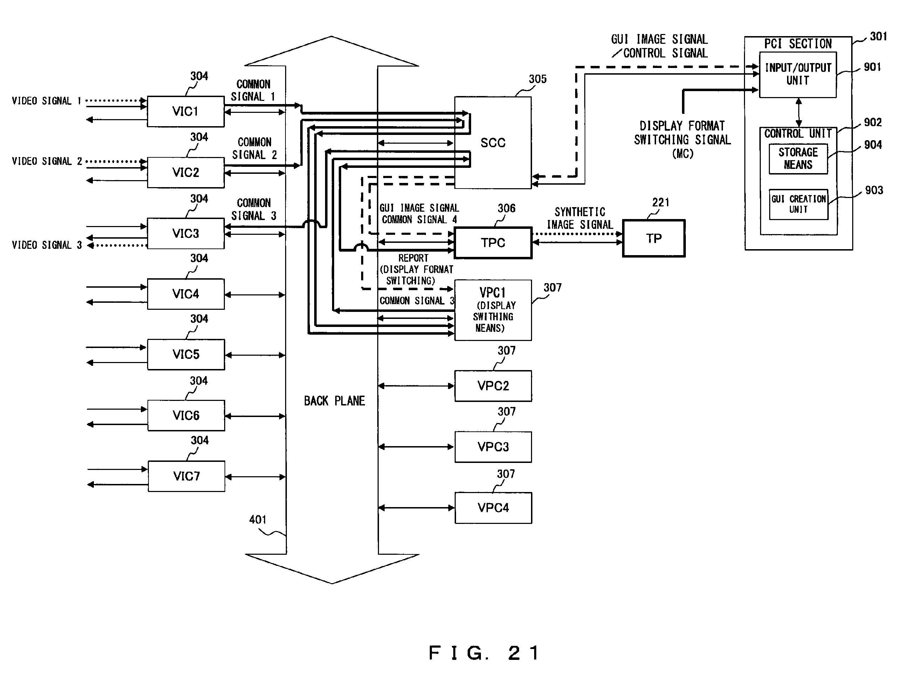

Fig. 21 shows a flow of respective image signals and control signals when images are edited.

The users select medical images to be edited by using the TP 221, an output-destination display device and a type of image processing. In Fig. 21, a medical image 1 and a medical image 2 transmitted from a plurality of medical devices (such as endoscopes) are selected, an output-destination display device 220 connected to a VIC3 304 is selected, and the PIP or the POP is selected as the image processing.

The medical images 1 and 2 are respectively input to the VIC1 304 and the VIC2 304 in the form of the video signals 1 and 2 (dotted lines), and are respectively converted into common signals 1 and 2 by the VIC1 304 and the VIC2 304.

The common signals 1 and 2 (represented by solid lines) are input into the SCC 305 via the back plane 401, and are input into the VPC1 307 via the prescribed path set by the SCC 305 in accordance with the setting made on an edition screen in the TP 221.

In the VPC1 307, image processing is performed on the common signals 1 and 2, and a common signal 3 (represented by a solid line) is output. Then, a GUI image is created in a GUI creation unit 903 in a control unit 902 in the PCI section 301, and is transferred to the SCC 305 via an input/output unit 901. The GUI image signal obtained in the SCC 305 is converted into a common signal 4, and is transferred to the TPC 306 via the back plane 401.

Synthetic image signals (represented by a dashed line) output from the TPC 306 are displayed in the TP 221 via the rear panel.

Next, the users view the synthetic images displayed on the TP 221, and determine the image (display format) . In the case of Fig.20, for example, respective display formats corresponding to the five images (A-E) are determined for each image. When the display format is determined by the TP 221, a determination signal is transferred to the input/output unit 901 in the PCI section 301. Thereafter, the control signal is transferred to the control unit 902. In the control unit 902, the display format and data corresponding to the display format are stored in the form of, for example, a table in a memory device (storage means 904). The determination signal includes data necessary to display the display format (information such as the type of the image processing, the image size, the position of the image, etc.).

Also, when determined, the determination signal is output from the TP 221 to the SCC 305, and in the SCC 305, the paths for the VPC1 304 and the VIC2 304 for inputting a video signal, and the output-destination VIC3 304 are formed, and the input/output path for the VPC1 307 is set.

Also, into the input/output unit 901 in the PCI section 301, the display format switching signal is transferred from the endoscope 1901. In accordance with the display format switching signal, the display format stored on the table in the control unit 902 is selected, and the selected display format is reported to the VPC 307 (display switching means 605) via the SCC 305. In accordance with this report, the type of a display format is set in the VPC 307, and images in the display format can be displayed on the display device.

The VPC 307 converts the synthetic image of the set display format into the common signal 3, and transfers it to the SCC 305. The SCC 305 transfers the common signal 3 to the VIC3 304, and the VIC3 304 is output to a desired display device.

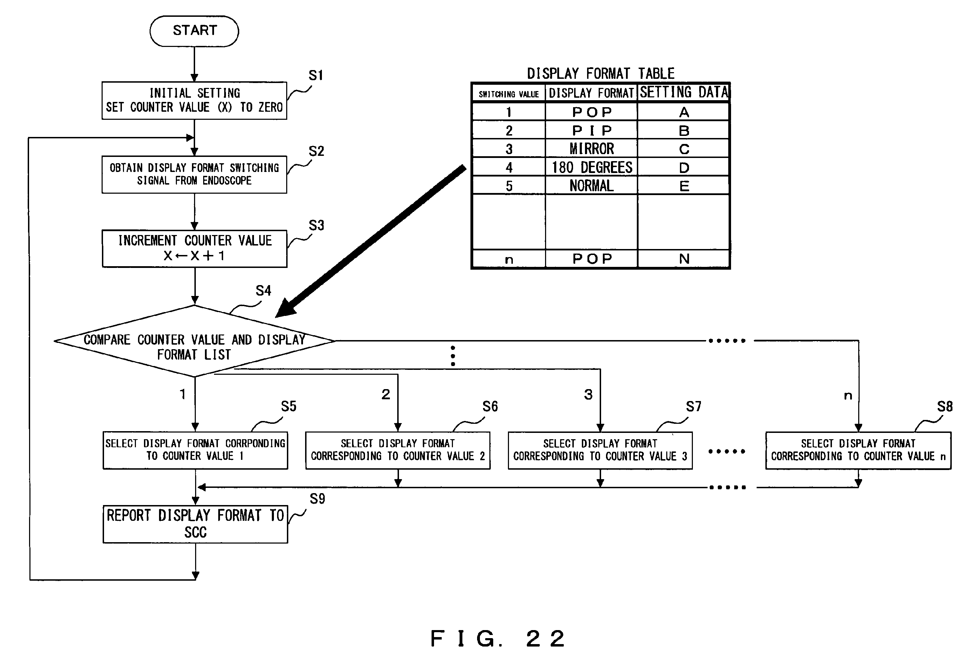

Fig. 22 is a flowchart for the switching process of display formats.

In step S1, the initial setting is performed. For example, the voltage level of the switching signal transferred from the MC is monitored, and when it has become "high", the value of a counter provided in the control unit 902 is incremented, and if a display format stored in advance is to be selected in accordance with the counter value, the counter value (X) is set to zero (X ← zero). In this example, a counter is used to perform the toggle switching; however, this is not intended to be limiting and it is also possible that switching can be performed from devices connected to the NMC202.

In step S2, the switching signal is obtained from the endoscope 1901. As explained in the above, the switching signal is obtained in the PCI section 301 via the MC 114.

In step S3, the counter value is incremented (X ← X+1). Detecting the change in the switching signal, the control unit 902 increments the counter value. The counter value returns to one when the value has reached the upper limit.

In step S4, the counter value and the switching value in the table (display format table) are compared to each other. When the comparison result is "1", the process proceeds to step S5. Similarly, the process proceeds to the respective steps in accordance with the counter value.

The table (display format table), as shown in Fig. 22, is set by the TP 221 in advance. The table shown in Fig. 22 stores "switching value", "display format", and "setting data". The "switching value" is a number assigned in accordance with the display format set by the TP 221, and corresponds to the counter value of the counter. The types of the image processing performed in the VPC 307 are stored as the "display format". The detailed setting for the image processing set as the display format is stored as the "setting data". Examples of the detailed setting are sizes of images, positions of images, and the like. Also, in the table (display image table), the "display format" and the "setting data" are separated; however, they may be processed together.

In step S5, the display format corresponding to the counter value 1 is selected, and "display format" and "setting data" are prepared as the display format switching signals.

In step S6, the display format corresponding to the counter value 2 is selected, and "display format" and "setting" data are prepared as another display format switching signals.

In step S7, the display format corresponding to the counter value 3 is selected, and "display format" and "setting" data are prepared as yet another display format switching signals.

In step S8, the display format corresponding to the counter value n (integer) is selected, and "display format" and "setting" data are prepared as still another display format switching signals.

In step S9, the display switching signals are transferred to the SCC 305.

In the present example, example 1, the display format is switched by using a switch device provided in the endoscope mainly; however, it is also possible for the display format to be switched by the TP 221.

According to the above configuration, it is possible to provide a medical support control system that controls medical devices and non-medical devices.

Conventionally, an operating person (such as a physician) has given instructions for changing display formats, and personnel such as nurses have had to edit a plurality of images by using the TP 221 in accordance with the instructions. Accordingly, troublesome manipulations have been required each time the display formats were to be switched. Also, sometimes it has been impossible to view necessary images at proper timings.

By employing the above configuration, necessary images can be viewed at proper timings. Also, it is possible to change the display formats from the sterilization area.

### (Variation Examples)

Display format tables are prepared respectively for procedures and medical instruments employed. Similarly to example 1, the display formats are stored in the storage means in a prescribed order. Because different patterns of image processing have to be used depending upon the procedures and medical instruments employed, they are stored in advance.

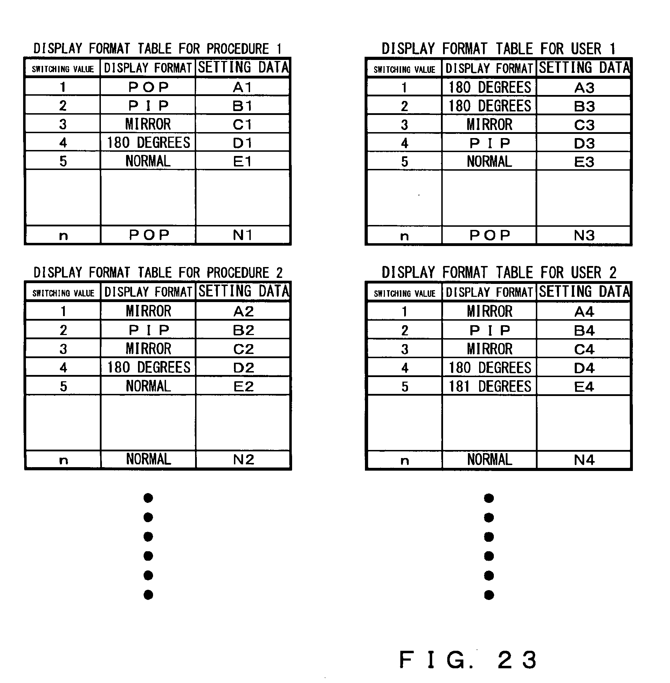

As shown in Fig. 23, a plurality of display format tables such as for procedures 1, 2, users 1, 2..., etc. are stored in the storage means. Users input the table contents through the TP 221 as in the example 1.

Then, when using the endoscopes, users select a table that is to be used at that time from the plurality of the display format tables by using the TP 221.

By preparing the display format tables as described above, it becomes unnecessary to perform setting for each of the procedures or users, which reduces the time used for the preparation for the operation.

The scope of the present invention is not limited to the above embodiments, and various alterations and modifications are allowed without departing from the spirit of the present invention.

### [Forth embodiment]

The VIC 304 has a function of inversely converting common signals into video signals appropriate to the output destination in the forth embodiment. Further, the respective VICs 304 can be inserted into a plurality of slots provided for the VICs 304. Also, all the VICs 304 can have a common interface connector. Also, when the power is in an off state, the VICs 304 perform switching to a path in which input video signals are directly output without being converted.

The SCC 305 selects the VIC 304 as the output destination in accordance with instructions given from the external environment in forth embodiment. Also, the SCC 305 obtains VIC-related information including identification information used for identifying the VICs 304 and position information specifying the positions of the corresponding VICs 304. The identification information is obtained from the VICs 304. Then, the SCC 305 detects, on the basis of the VIC-related information, the position of the VIC 304 as the output destination set in accordance with the instruction given from the external environment, and selects the VIC 304 or the VPC 307 as the output destination for the common signal.

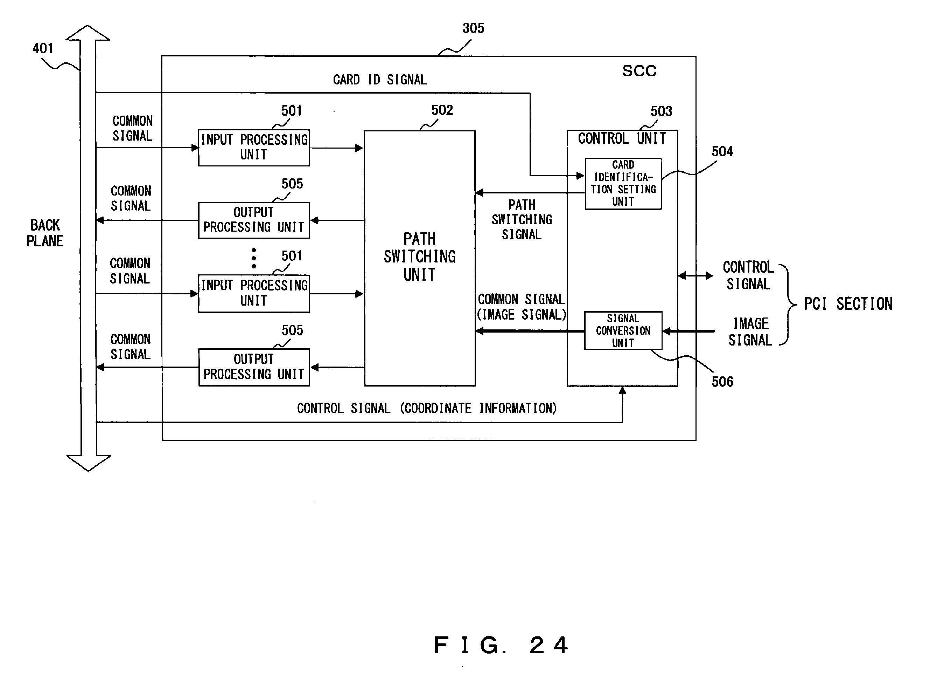

Fig. 24 shows a configuration of the VIC 304 in forth embodiment.

The VIC 304 is attached to a back plane 401, and includes an input processing unit 402, a signal conversion unit 403, and an output processing unit 404. In this configuration, the back plane 401 includes slots into which the audio interface cards (AIC) 303, the video interface cards (VIC) 304, the switching control card (SSC) 305, a touch panel card (TPC) 306, and the video processing cards (VPC) 307 are inserted. These cards perform communications via the back plane 401.

The input processing unit 402 receives the video signals output from devices (medical devices and non-medical devices) that are connected to the MC114 and the NMC 202 and are used for outputting video signals, and transfers the received video signals to the signal conversion unit 403.

The path switching unit 502 determines the paths for the common signals to be transmitted to the output-destination VIC 304 in accordance with the path switching signals output from the control unit 503. Also, it determines the path for the common signals to be transmitted to the output-destination VPC 307 in accordance with the path switching signal when image processing is to be performed in the VPC 307. Also, it determines the path for the common signals to be transmitted to the VIC 304 after the image processing in the VPC 307.

The control unit 503 has a card identification setting unit 504 and a signal conversion unit 506, transfers the control signals input from an external connection device to the PCI section 301, and obtains the control signal input from the PCI section 301 in order to control the respective units in the SCC 305.

The signal conversion unit 506 obtains image signals input through the PCI section 301, and converts the signals into common signals in order to transfer the converted signals to the path switching unit 502.

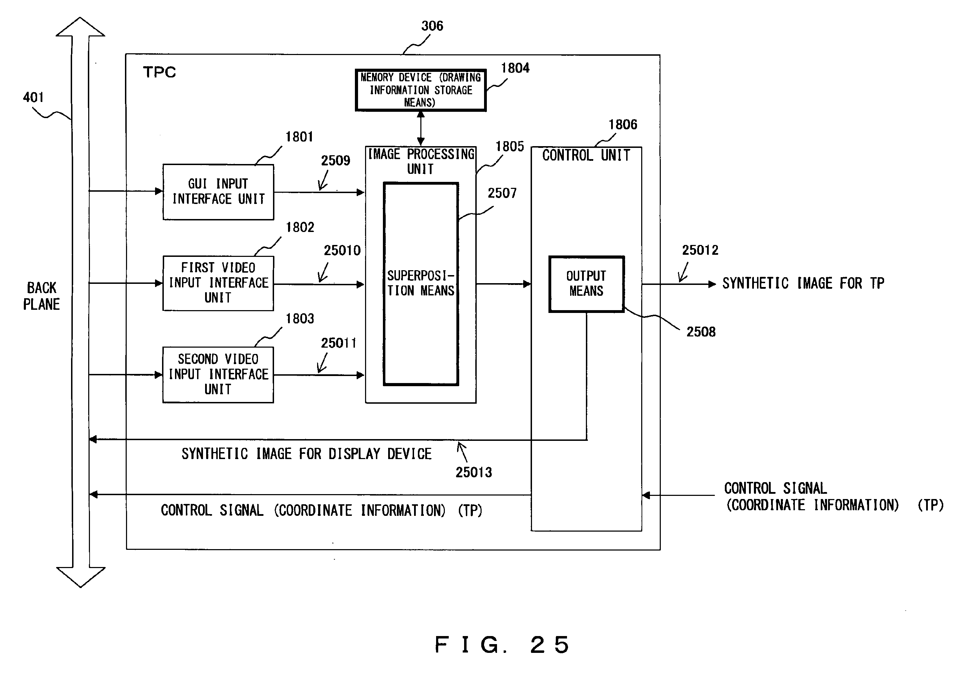

Fig. 25 shows a configuration of the touch panel card 306.

The TPC 306 is included in the back plane 401, and has a function of performing image processing in accordance with instructions given from the TPs 113 and 221.

The TPC 306 includes a GUI input interface unit 1801, a first video input interface unit 1802, a second video input interface unit 1803, a memory device 1804 (drawing information storage unit), an image processing unit 1805, and a control unit 1806.

The GUI input interface unit 1801 is an interface used for obtaining, via the SCC 305 and the back plane 401, window layout information (hereinafter, referred to as a GUI (Graphical User Interface) window) created in the PCI section 301, and for outputting the information to the image processing unit 1805.

The first video interface unit 1802 and the second video interface unit 1803 obtain a medical image from the medical device group 160, and output the obtained data to the image processing unit 1805. In this configuration, the medical images are images obtain by endoscopes 146 and 147 or by other medical devices (such as an X-ray imaging machine).

In this configuration, the medical images are input into the VIC 304 as video signals, are converted into common signals in the VIC 304, and are input into the SCC 305. Thereafter, the medical images that have been converted into the common signals are output to the output-destination VPC 307 in accordance with the setting in the SCC 305, undergo image processing in the VPC 307, and are input into the first video input interface unit 1802 and the second video input interface unit 1803.

The memory device 1804 stores the GUI images (image signals) obtained through the GUI input interface unit 1801, the medical images obtained through the first video interface unit 1802 or the second video interface unit 1803, or the images processed in the image processing unit 1805. Also, it stores drawing information (that will be referred to later) that is transferred together with the GUI images.

Also, when a portion of the GUI image overlaps with the image to be output to the display device 220, the overlapping portion of the GUI image is deleted, and the image to be output to the display device 220 is stored. An example of an overlapping portion of the GUI image is a menu bar.

The image processing unit 1805 performs image processing on the respective images obtained through the GUI input interface unit 1801, the first video input interface unit 1802, and the second video input interface unit 1803, and transfers the image-processed images to the control unit 706. Also, it includes the medical images in a prescribed region in the GUI images, and creates synthetic images by synthesizing the GUI images and the medical images.

Superposition means 2507 creates a superposition image by superposing the drawing information on the medical images.

The control unit 1806 directly outputs the images that were image-processed by the TP 221. Also, the control unit 1806 is a device used for controlling the entirety of the TPC 306.

Output means 2508 outputs the synthetic images for the display device to the display device 220, and outputs the synthetic images for the TP to the TPs 113 and 221.

Also, the superposition means 2507 and the output means 2508 may be provided in different blocks from the blocks for the image processing unit 1805, and the control unit 1806.

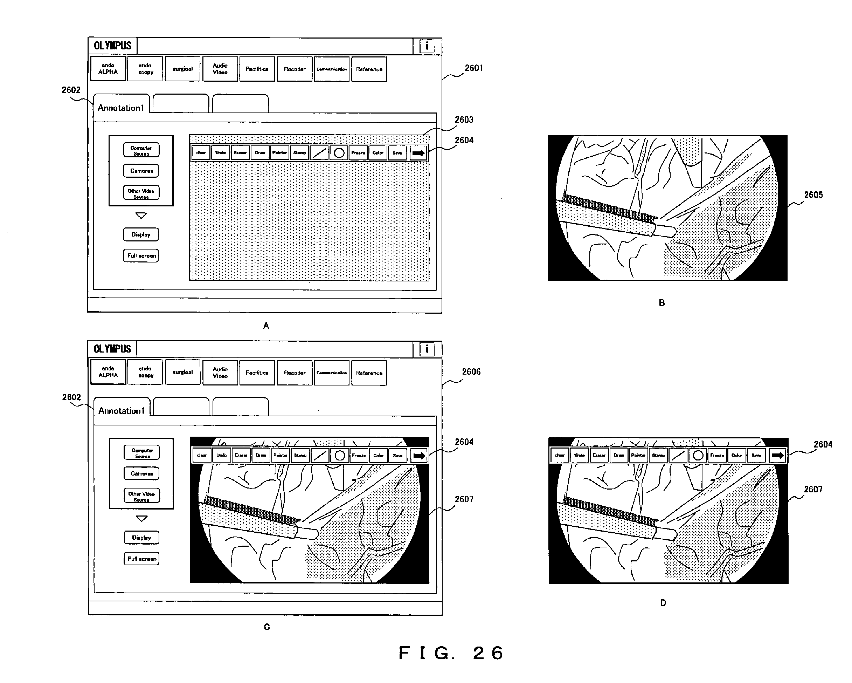

A GUI image 2 601 (Fig. 26A) is an image of an output 2509 of the GUI input interface unit 2601. In the example of Fig. 26, the GUI image 1801 has an "Annotation" selection switch 2602 used to select an edit window (for example, a superposition image creation window), an image region 2603 for outputting medical images, and an edit operation selection section 2604 that is a "menu bar" for selecting an edit operation of the medical devices.

A medical image 2605 (Fig. 26B) is an image of an output 25010 of the first video input interface unit 702. Amedical image 2605 is a single medical image input from the VIC 304. Also, the medical image may be an image that was image-processed by the VPC 307.

A synthetic image 2606 for the TP (Fig. 26C (GUI image + medical image)) is an image of an output 25012 from the control unit 1806, and is an image created by synthesizing the GUI image 1801 obtained through the GUI input interface unit 2601 and the medical image 2605 obtained through the first video input interface unit 1802. The synthetic image 2606 is displayed in the TPs 113 and 221.

A synthetic image 2607 for the display device (Fig. 26D (medical image + image)) is an image of an output 25013 of the output means 2508, and is an image to be output to the display device 220.

In this configuration, the edit operation selection section 2604 has selection switches such as "clear", "undo", "eraser", "draw", "pointer", "stamp", "straight line", "circle", "freeze", "color", "save", or the like. Pressing "Clear" clears all the drawing images. Pressing "undo" returns what was done to the previousstate. Pressing "eraser" erases only a portion of the selected drawing image. Pressing "draw" performs drawing by following a trace of the pointer. Pressing "pointer" changes the thickness of the lines drawn. Pressing "stamp" draws a time stamp or figures peculiar to users. Pressing "straight line" draws a line between two points selected. Pressing "circle" draws circles or ovals. Pressing "freeze" fixes the image. Pressing "color" changes the colors of the lines or circles drawn. Pressing "save" saves the current image.

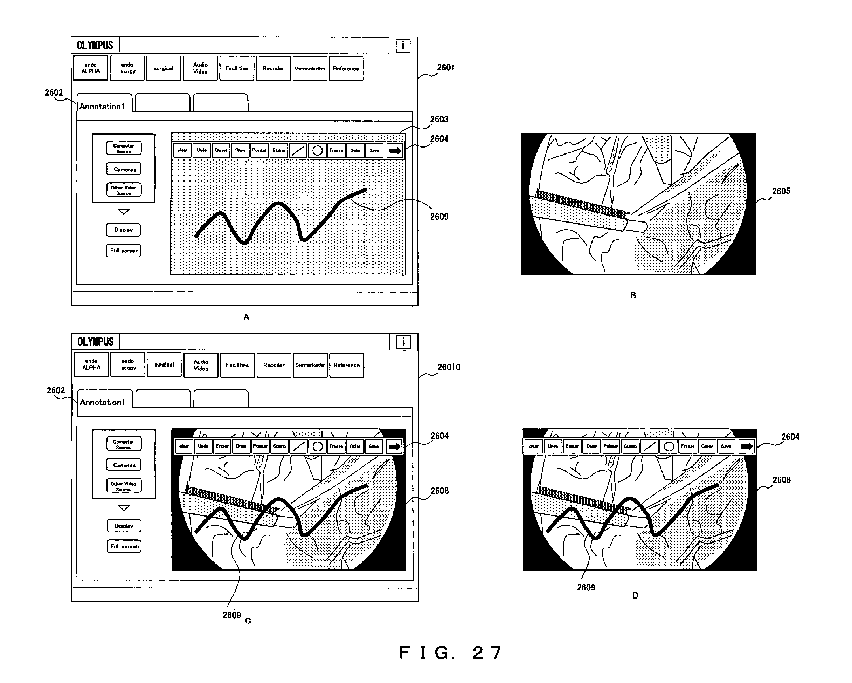

The GUI image 2601 (Fig. 27A) is an image of the output 2509 of the GUI input interface unit 1801. In the example of Fig. 27, the GUI image 2601 has the "Annotation" selection switch 2602 for selecting the edit window, the image region 2603 for outputting the medical images, and the edit operation selection section 2604 that is the menu bar for selecting the manner of editing the medical images. Also, a drawing image 2609 is displayed in the image region 2603. The drawing image 2609 is a figure drawn by using the function of the edit operation selection section 2604.

The drawing image 2609 is drawn by the users through the TPs 113 and 221, and the information (coordinate information) of the drawing image is transferred to the PCI section 301. Then, in the PCI section 301, a drawing image (drawing information) is created on the basis of the information, and the drawing image 2609 is displayed after being transferred to the TPC 306 together with the GUI images.

Fig. 27B shows the same image as that shown in Fig. 26B, and it is an image of the output 25010 from the first video input interface unit 1802. Also, the medical image 2605 is a single medical image input from the VIC 304. Also, the medical image may be an image that was image-processed by the VPC 307.

Fig. 27C shows an image of the output 25012 of the control unit 1806, and is an image created by synthesizing the GUI image 2601 obtained through the GUI input interface unit 2601 and the medical image 2605 obtained through the first video input interface unit 1802 by using the image processing unit 1805 or the like.

Also, Fig. 27C shows an image displayed in the TPs 113, 221, or the like. In the image region 2603, the superposition image 2608 obtained by superposing the medical image 2605 and the drawing image 2609 is displayed. Also, the edit operation selection section 2604 is displayed on the superposition image 2608.

Fig. 27D shows an image to be output to the display device 220 (superposition image 2608 (Fig. 27D (medical image + drawing image)) and the edit operation selection section 2604), and is an image of the output 25013 of the output means 2508.

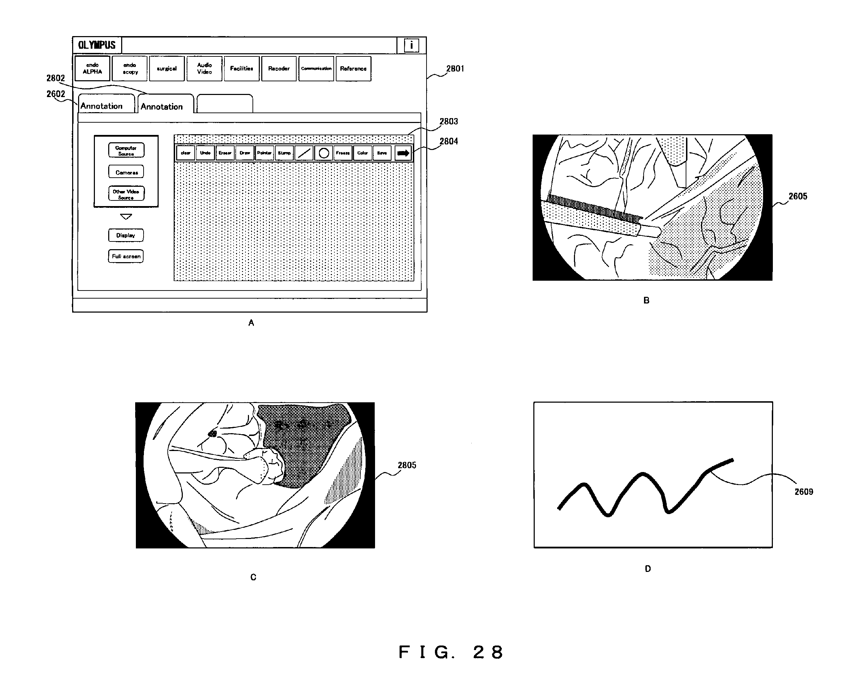

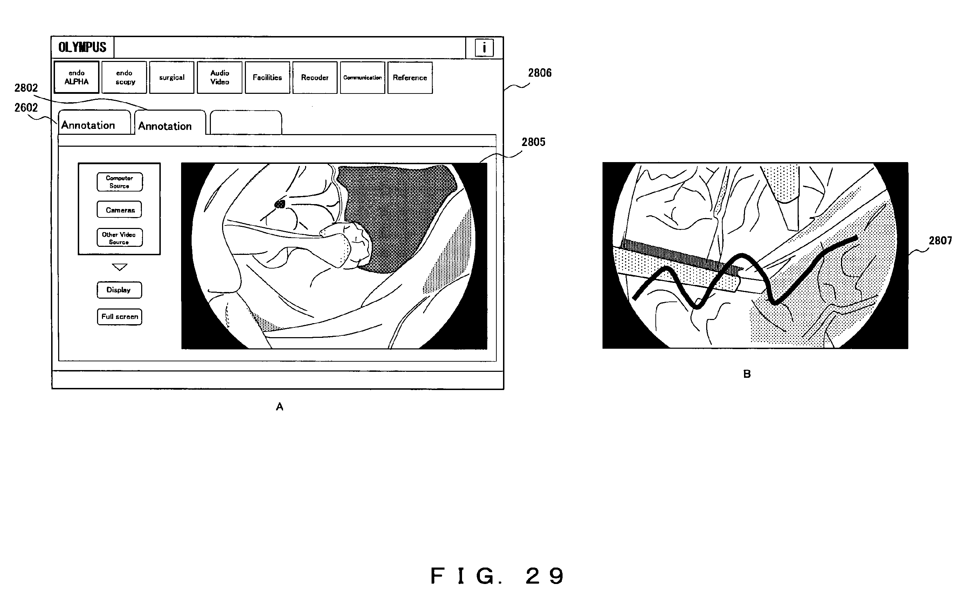

Figs. 28 and 29 show a case when another window is opened.

As an example, a case is shown in which the edition of the GUI image 2601 ("Annotation": superposition image creation window) shown in Fig. 27C is completed, and a GUI image 2601 ("recording device manipulation window") that is the next window shown in Fig. 28A is displayed. Also, a case is shown in which the image 2605 shown in Fig. 28B is changed to a medical image 2805, shown in Fig. 28C.

The GUI image 2801 is an image of the output 2509 of the GUI input interface unit 1801. In the example of Fig. 28, the GUI image 2801 has a "Setting Recorder" selection switch 2802 used for selecting operation windows, an image region 2803 used for outputting medical images, and an edit operation selection section that is a "menu bar" used for selecting an edit operation of the medical images.

Fig. 28B shows the same image as is shown in Fig. 26B, and is an image of the output 25010 of the first video input interface unit 1802. Also, the medical image 2805 is a single medical image input through the VIC 304.

The image 2805 shown in Fig. 28C is an image of an output 25011 of the second video input interface unit 1803. Also, the medical image 2805 is a single medical image input through the VIC 304. Also, the medical images may be images that were image-processed by the VPC 307.

The drawing image 2609 shown in Fig. 28D is a drawing image drawn by a user by using a device such as the TPs 113 or 221 before performing image processing by using the GUI image 2801. The drawing information of the drawing image is held in the memory device 1804 (drawing information storage means).

A synthetic image 2806, shown in Fig. 29A, is an image of the output 25012 of the control unit 1806, and is created by synthesizing, by using the image processing unit 1805, the GUI image 2801 obtained through the GUI input interface unit 1801 and the medical image 2805 obtained through the second video input interface unit 1803.

The synthetic image 2806 shown in Fig. 29A is displayed in the TPs 113 and 221 or the like, and the medical image 2805 is displayed in the image region 2803.

Fig. 29B shows an image to be output to the display device 220. The image 2807 is an image of the output 25013 from the output means 2508. It is an image obtained by superposing the medical image 2605 obtained through the first video input interface unit 1802 and the drawing image 2609 stored in the memory device 1804.

As described above, by using the TPC 306, even when the TP 113 or 221 displays a different window, the superposition images whose edit is currently being performed or whose edit has been completed can be displayed.

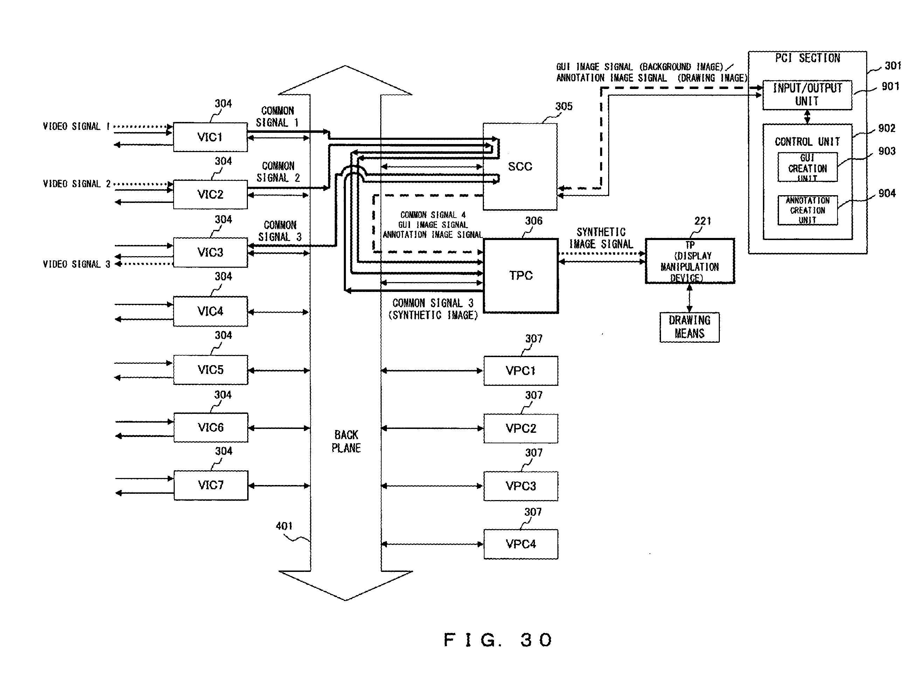

Fig. 30 shows a flow of signals of the respective image signals when an image edit is performed.

The users select a medical image to be edited by using the TP 221 and an output-destination display device (such as the display device 220).

In Fig. 30, a medical image 1 that is transmitted from a plurality of the medical devices (such as the endoscopes) is selected, and an output-destination display device connected to the VIC3 304 is selected.

For example, it is assumed that the GUI image to be displayed on the TP 221 is the GUI image 2601, and that the selected medical image 1 is the medical image 2605. The medical image 2605 is displayed in the image region 2603 (Fig. 26A: Annotation) in the display window of the TP 221.

The medical image 1 is input to the VIC1 304 as a video signal 1 (represented by a dotted line), and is converted into a common signal 1 in the VIC1 304. The common signal 1 (represented by a solid line) is input into the SCC 305 via the back plane 401, and thereafter is input into the TPC 306.

The GUI images are created in a GUI creation unit 903 in the control unit 902 (such as the CPU or the like) in the PCI section 301, and are transferred to the SCC 305 via an input/output unit 901. The GUI image obtained in the SCC 305 is transferred to the TPC 306 via the back plane 401.

The GUI image 2601 and the medical image 2605 are synthesized by the TPC 306, and the synthetic image 806 shown in Fig. 26C is displayed in the TP 221. Also, the image 2607 in the image region 2603 of the synthetic image 2606 is converted into a common signal 3, and is output to the VIC3 304 via the SCC 305. In this configuration, the edit operation selection section 2604 is included in the output synthetic image 2607.

Next, the user performs drawing on the medical image 2605 by using drawing means (such as a mouse) in the TP 221, and the synthetic image 2606 shown in Fig. 27C is displayed on the TP 221.

The drawing image 2609 displayed in the synthetic image 2606 is created in an annotation creation unit 904 in the control unit 902 (such as the CPU or the like) in the PCI section 301 on the basis of the coordination information transmitted from the TP 221 to the PCI section 301. Then, the created drawing image 2609 is transmitted as the GUI image signal/annotation image signal to the SCC 305 via the input/output unit 901 together with the GUI image 2601 (including the edit operation selection section 2604). The GUI image signal/annotation image signal obtained in the SCC 305 is transferred to the TPC 306 via the back plane 401.

The superposition images 2608 obtained by superposing the drawing image 2609 on the GUI image 2601 and on the medical image 2605 are synthesized by the TPC 306, and a synthetic image 26010 shown in Fig. 27C is displayed in the TP 221.

The superposition image 2608 of the image region 2603 of the synthetic image 26010 is converted into a common signal 3 (represented by a solid line), and is output to the VIC3 304 via the SCC 305. In this configuration, the edit operation selection section 2604 is included in the output synthetic image 26010.

Next, from the edit using the GUI image 2801, the GUI image 2601 (recording device manipulation window) shown in Fig. 28A is displayed

In Fig. 30, a medical image 2 that is transmitted from a plurality of the medical devices (such as the endoscopes) is selected.

For example, it is assumed that the GUI image to be displayed on the TP 221 is the GUI image 2801, and the selected medical image 2 is the medical image 2805. The medical image 2805 is displayed in the image region 2803 (Fig. 29A: recording device manipulation window) in the display window of the TP 221.

The medical image 2 is input into the VIC2 304 as a video signal 2 (represented by a dotted line), and is converted into a common signal 2 in the VIC2 304. The common signal 2 (represented by a solid line) is input into the SCC 305 via the back plane 401, and thereafter is input into the TPC 306.

The GUI image 2801 is created in the GUI creation unit 903 in the control unit 902 (such as the CPU or the like) in the PCI section 301, and is transferred to the SCC 305 via the input/output unit 901. The GUI image signals obtained in the SCC 305 is transferred to the TPC 306 via the back plane 401.

The GUI image 2801 and the medical image 2805 are synthesized by the TPC 306, and a synthetic image 2806, shown in Fig. 29A, is displayed in the TP 221.

Also, when an edit is being performed by using the synthetic image 2606, the image 2807 shown in Fig. 29B made using the medical image 2605 and the drawing image 2609 stored in the memory device 1804 is converted into the common signal 3 (represented by a solid line), and is output to the VIC3 304 via the SCC 305.

The image 2807 is output in a state in which the edit operation selection section 2604 is erased from the superposition image 2608 in Fig. 27.

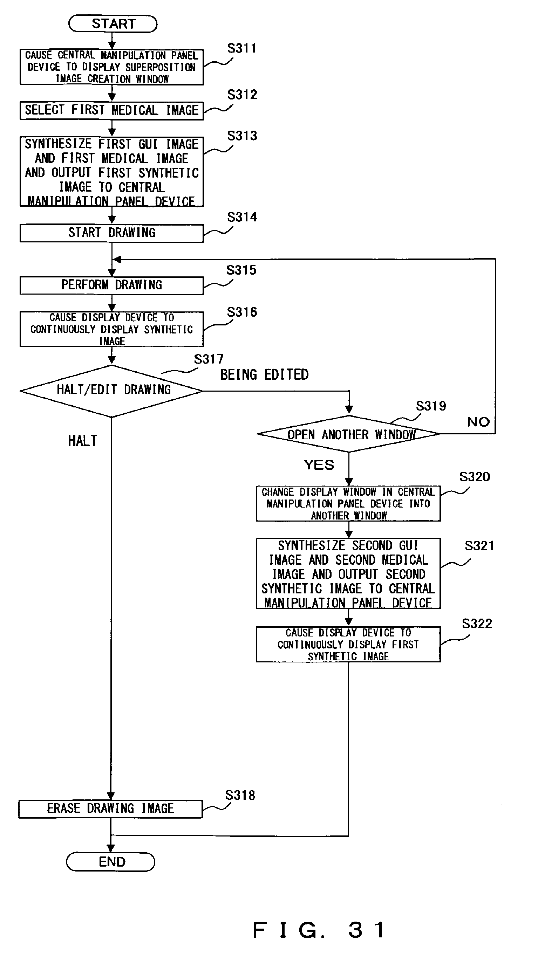

Fig. 31 is a flowchart for a process of synthesizing the GUI image obtained through the GUI input interface unit 1801 and the medical image obtained through the first video input interface unit 1802 and the second video input interface unit 1803.

In step S311, the display window in the TP 221 displays an edit window (such as the superposition image creation window). For example, users perform editing while viewing the first GUI image (GUI image 2601) shown in Fig. 26 that is displayed in the TP 221 or the synthetic image 2606. In this configuration, the transition to the edit window is made when the "Annotation" selection switch 2602 shown in Fig. 26 is selected.

In step S312, the firstmedical image (medical image 2605) to be displayed in a plurality of display devices is selected. For example, an image of the corresponding endoscope is displayed.

In step S313, a first synthetic image (synthetic image 2606) obtained by synthesizing the first GUI image and the first medical image by using the TPC 306 is output to the TP 221.

In step S314, drawing starts. For example, the edit operation selection section 2604, i.e., a "menu bar", for selecting the edit operation on medical images is displayed.

In step S315, drawing is performed. For example, a drawing image such as the drawing image 2609 shown in Fig. 27A is displayed. Then, the superposition image 2608 shown in Fig. 27C is displayed in the TP 221. When the edit on the superposition image 2608 performed by the user is completed, the superposition image 2608 is displayed in the display device 220.

In step S316, the display device 220 continuously displays the superposition image 2608.

In step S317, it is determined whether the drawing was halted or has continued. When it is determined that the drawing was halted, the process proceeds to step S318. Further, when another window is to be opened, the process proceeds to step S319.

In step S318, the drawing image is erased. For example, the drawing image 2609 is deleted.

In step S319, it is determined whether or not another window was opened. When a transition to another window is to be made, the process proceeds to step S320. Further, when an edit is to be performed on the first synthetic image, the process proceeds to step S315.

In step S320, the TP 221 displays the second GUI image, which is another window. For example, the GUI image 2801 as shown in Fig. 28A is opened. The second GUI image is displayed when the "Setting Recorder" selection switch 2802 is selected.

In step S321, the second synthetic image (synthetic image 2806) obtained by synthesizing the second GUI image and the second medical image by using the TPC 306 is output to the TP 221. A second medical image (medical image 2805) to be displayed in a plurality of display devices is displayed. For example, an image of the corresponding endoscope is displayed.

In step S322, the display device 220 is caused to continue to display the superposition image 2608.

Conventionally, the superposition images were displayed only in the TP 221. However, in the present invention such images are also displayed in the display device 220 or the like. Also, it is possible to distribute the superposition images not only to the display device 220, but also to a plurality of other display devices.

Further, conventionally, when the edit window (such as the superposition image creation window or the like) in which a superposition image is being created transits to another window, the superposition image that is in the display device 220 was deleted; however, in the present invention the superposition image can be continuously displayed in the display device 220 even when the edit window transits to another edit window.

Also, when an edit window in which a superposition image is being edited has transited to another edit window, it has conventionally not been able to pre-view the medical images or the like to be edited in the other edit window. However, in the present invention it is possible to display the medical images to be edited in the TP 221 while it is being edited.

The scope of the present invention is not limited to the above embodiments, and various alterations and modifications are allowed without departing from the spirit of the present invention.

### [Fifth embodiment]

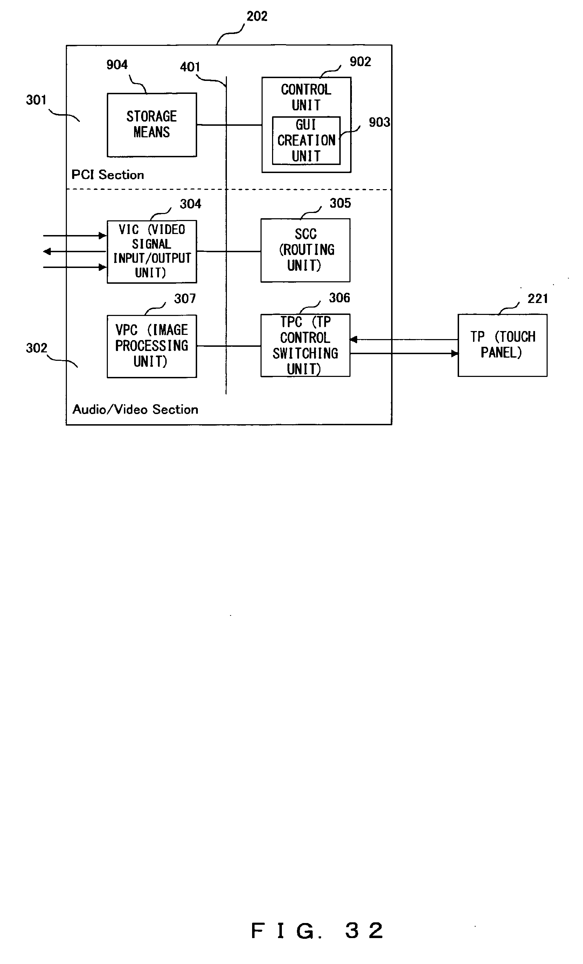

Fig. 32 is a block diagram showing a configuration of the NMC 202 in the present embodiment. The NMC 202 includes a PCI section 301 and an audio/video (A/V) section 302.

The PCI section 301 mainly controls a non-medical device group 210 connected to the NMC 202. The PCI section 301 includes a control unit 902 and a storage device 904. The control unit 902 controls the entirety of the PCI section 301, and transmits and receives data to and from the A/V section 302. The control unit 902 includes a GUI creation unit 903 and the like. Numeral 3301 denotes a back plane.

The GUI creation unit 903 creates Graphical User Interface image information (hereinafter, referred to as GUI image information) that is an image layout to be displayed on a TP 221 or a monitor device, and transmits it to a SCC 305 (routing unit).

The storage device 904 stores various programs, information set by the TP 221, and the like.

The A/V section 302 is a section that mainly processes the image signals and the audio signals. The A/V section 302 includes VIC 304 (a video signal input/output unit), VPC 307 (an image processing unit), SCC 305 (a routing unit), and TPC 306 (a TP control unit).

The VIC 304 has a plurality of video signal input ports and a plurality of video signal output ports.

The SCC 305 switches routes for the video signals input from the VIC 304 and the video signals that were processed in the VPC 307, and transfers them to a prescribed configuration unit in the NMC 202. Also, the SCC 305 transfers the GUI image information created in the GUI creation unit 903 to the TPC 306.

The VPC 307 performs image processing on the image information transferred from the SCC 305. Examples of the image processing are the enlargement/reduction (scaling) of images, the mirroring of images, the rotation of images, displaying another, smaller image in a main image (picture in picture (PIP)), and displaying a plurality of images side by side (picture out picture (POP)).

The TPC 306 synthesizes the GUI image created in the GUI creation unit 903 with images created on the basis of the video signals transmitted from the VIC 304. Then, the TPC 306 outputs the synthesized image to the TP 221. Also, the TPC 306 receives control signals (coordinate information) on the basis of touch manipulations from the TP 221, and transfers them to the control unit 902. Further, the TPC 306 can perform switching between the NMC 202 and the MC 114 as the manipulation targets of the TP 221.

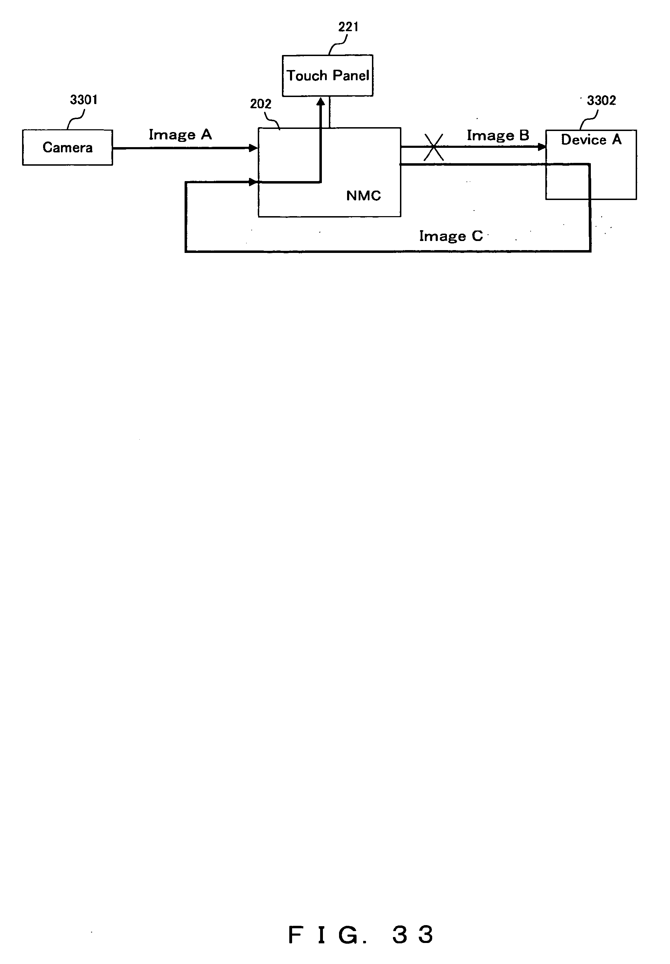

Fig. 33 shows the principle for performing a preview of video signals looped back to the NMC 202 according to the present embodiment.

When, for example, image A obtained from a camera 3301 is recorded on device A (3302), image A that is the recording target is selected by using the TP 221, and device A (3302) is selected as the output destination of image A (image B represents the image output from the NMC 202 to device A). In this case, the TP 221 performs a preview of image A for the confirmation of selection of image A.

When a cable between the NMC 202 and device A (3302) is disconnected or when a connector thereof is removed, image B is not output to device A. However, a preview image of image A is displayed on the TP 221. This misleads the users into believing that image A in the preview image has been recorded on device A.

Accordingly, the image displayed on the preview area on the window displayed on the TP 221 is output via device A. In other words, when device A is selected as the recording destination of the video signals, an image returned from device A (image C) is designed to be automatically displayed as an image for the preview image to be displayed on the TP 221.

As will be explained by referring to the next figure, in the present embodiment, on the basis of the routing information set in advance, it is possible that the video signals input into the NMC 202 are output to a prescribed device and are recorded; thereafter the video signals are returned to the NMC 202 (in other words, the video signals are looped back), and are output to the TP 221.

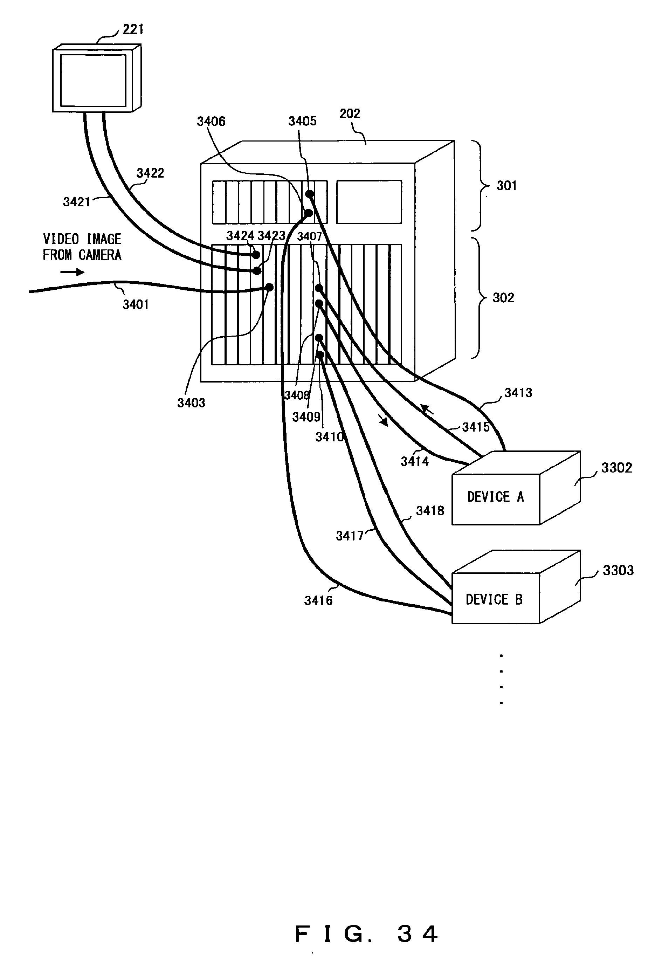

Fig. 34 shows an example of connection between the NMC 202 and the recorder for performing the preview of the video signals from the endoscope looped back from the recorder according to the present embodiment.

A video signal input port 3403 of the NMC 202 is connected to the camera head of the endoscope via a communication cable 3401. The video signals obtained through the endoscope are input into the video signal input port 3403 via the communication cable 3401.

Video signal output ports 3408 and 3410 of the NMC 202 and video signal output ports of device A (3302) and device B (3303) are respectively connected to each other via image cables 3414 and 3417. Video signal input ports 3407 and 3418 of the NMC 202 and video signal output ports of device A (3302) and device B (3303) are respectively connected via image cables 3415 and 3418. Also, communication ports 3405 and 3406 on the PCI section 301 side of the NMC 202 are respectively connected to communication ports of device A (3302) and device B (3303) via communication cables 3413 and 3416. Further, a plurality of devices may be connected.

Cables 3421 and 3422, which extend from the TP 221, are respectively connected to a TP output port 3423 and a TP input port 3424 of the NMC 202. The cable 3421 is a cable for transmitting to the TP 221 images synthesized in the TPC 306. The cable 3422 is a cable for transmitting to the TPC 306 the control signal based on touch manipulations on the TP 221.

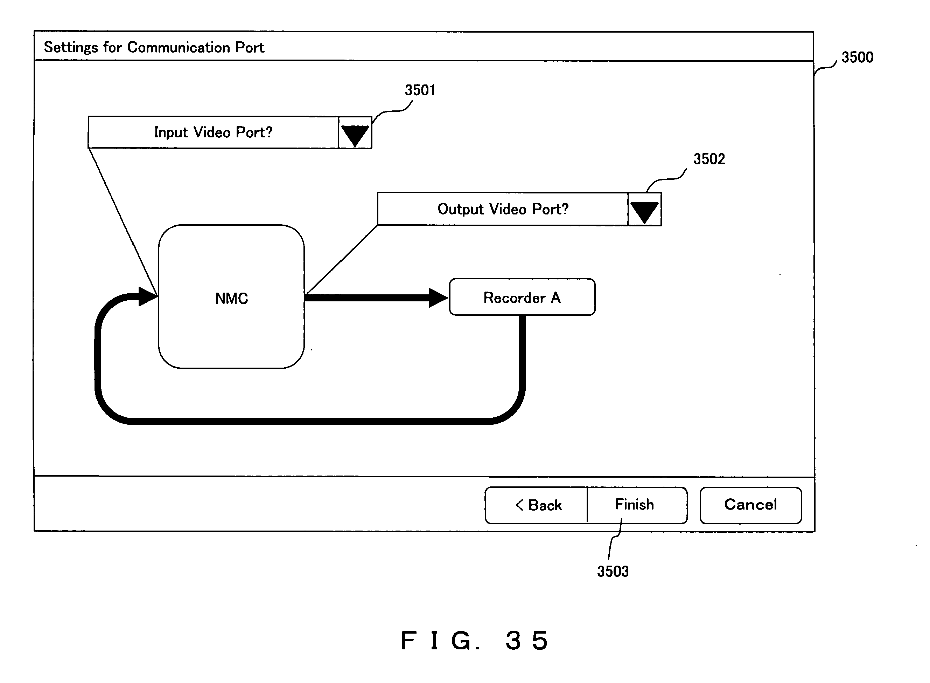

Fig. 35 shows a communication port setting window 3500 for device A according to the present embodiment. The communication port setting window 3500 is displayed on the TP 221 in the administrator mode. On the communication port setting window 3500, it is possible to perform setting for the looping back of video signals for each device.

In an output video port setting unit 3502, it is possible to set the output port on the NMC 202 side from which the video signals output to the device A. In Fig. 34, the video signal output port 3408 is set in the output video port setting unit 3502.

It is possible to set the input port on the NMC 202 side into which video signals output from device A are input. In Fig. 34, the video signal input port 3407 is set in an input video port setting unit 3501.

The information set in the communication port setting window 3500 is registered in the storage device 904 when a "Finish" button 3503 is pressed.

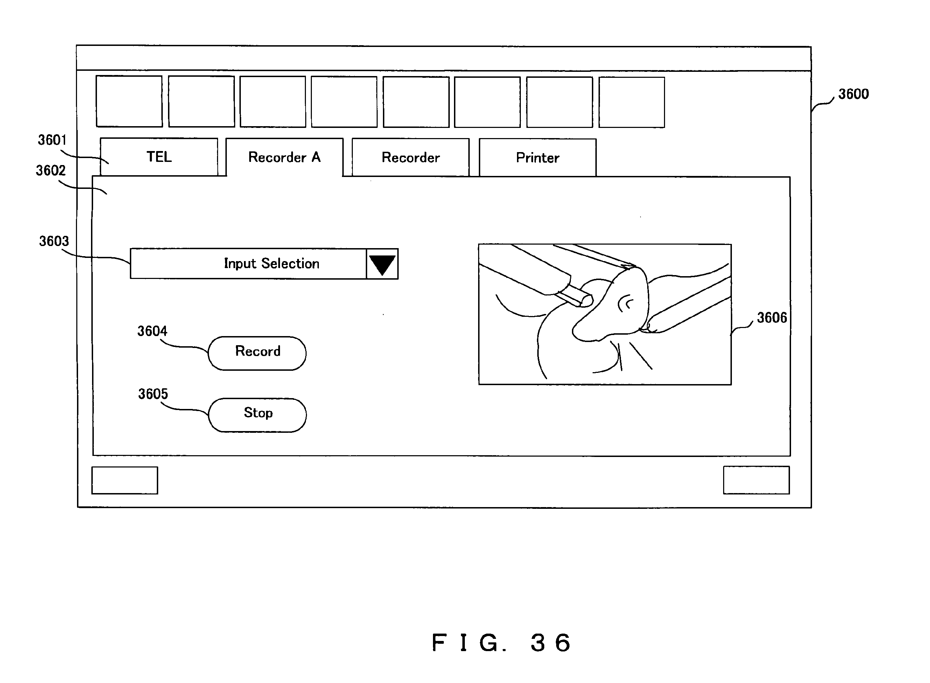

Fig.36 shows an operation window 3600 for device A according to the present embodiment. The operation window 3600 is displayed on the TP 221. The operation window 3600 includes tabs 3601, a selection tab display area 3602, an input video selection area 3603, a record button 3604, a stop button 3605, and a preview area 3606.

On the operation window 3600, it is possible to perform operations for the device selected through the selection of the tabs 3601. When one of the tabs 3601 is selected, the selection tab display area 3602 is displayed. In Fig. 36, the tab "Recorder A" is selected, and a selection tab display area 3611 for the Recorder A is displayed. Accordingly, in the example of the operation window 3600 shown in Fig. 36, it is possible to manipulate the Recorder A. Also, in the example of the present embodiment, device A is Recorder A; however, the scope of the present invention is not limited to this example, and device A can be various devices.

In the input video selection area 3603, it is possible to select an input source (such as an endoscope or the like) of video signals to be output to the device selected in the tag 3601.

In the preview area 3606, the image selected in the input video selection area 3603 is displayed. However, as will be described later, when looping back is set in the setting window shown in Fig. 35, the signals of the image displayed in the preview area 3606 are not the video signals output from the input source to the TP 221 via the NMC 202, but the video signals via device A.

When the record button 3604 is pressed, the image displayed in the preview area is recorded on the device selected via the tabs 3601. In this case, the image can be recorded on the "Recorder A". When a stop button 3605 is pressed, it is possible to stop the recording operation.

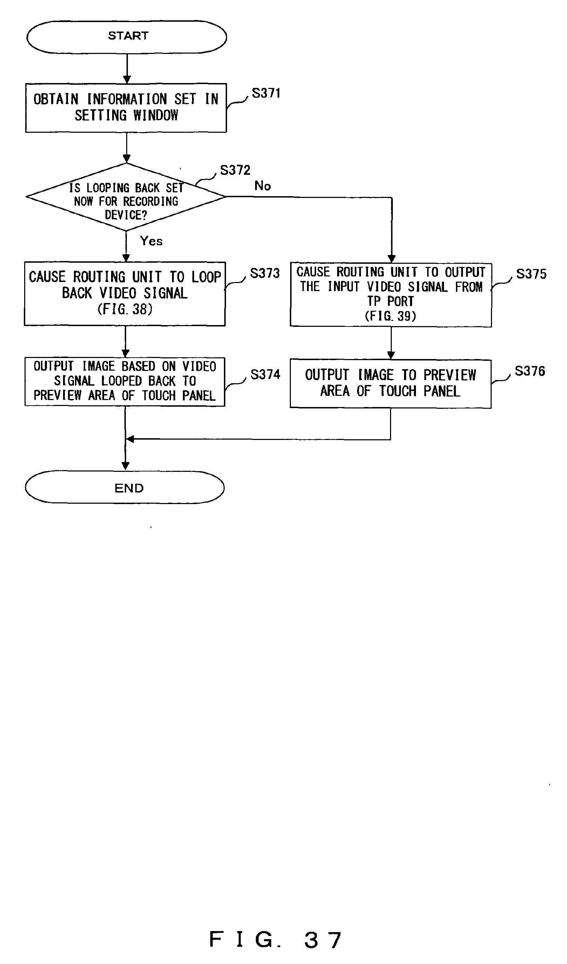

Fig. 37 is a process flow for the control unit based on the loop-back setting according to the present embodiment. When the tab 3601 and the input video selection unit 3603 are selected on the operation window 3600, the control unit 902 executes the processes in the flowchart shown in Fig. 37.

First, using the tab "Recorder A" selected in the operation window 3600 as the key, the control unit 902 searches for the setting information for device A, which is the manipulation target device, fromamonginformation set in the communication port setting window 3500 stored in the storage device 904, and reads the setting information (S371).

Next, on the basis of the read setting information about device A, the control unit 902 determines whether or not looping back is set for device A (S372).

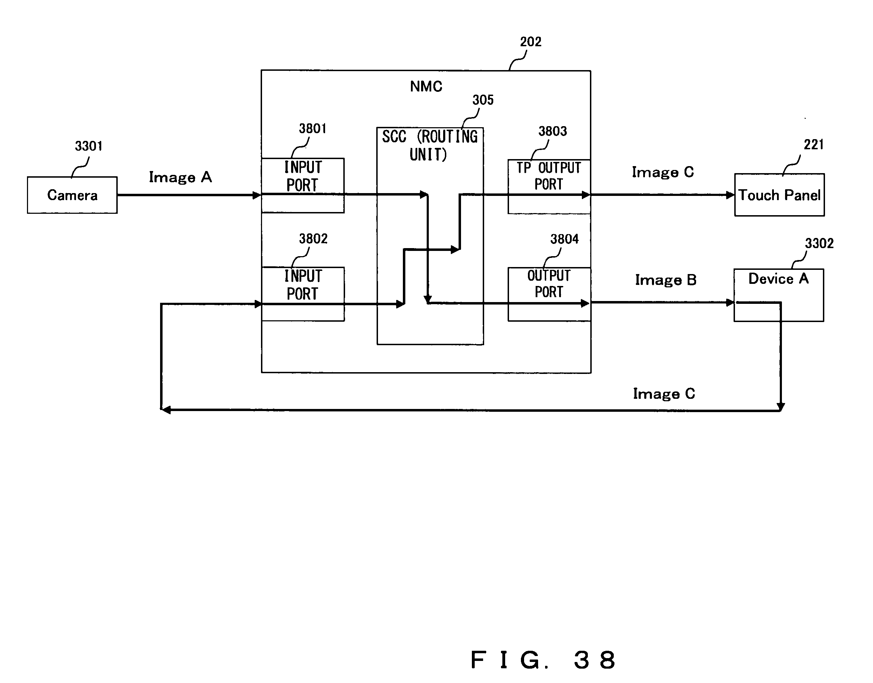

When it is found that looping back is set in S372 (Yes in S372), the control unit 902 causes the SCC 305 to perform routing of video signals, and causes the video signals to loop back (S373). This will be explained in Fig. 38.

Fig. 38 is a flowchart for the video signals when looping back is set according to the present embodiment. The image signal (image A) output from the camera head of the endoscope (camera 3301) is input into the NMC 202 via an input port 3801. In accordance with instructions from the control unit 902, the SCC 305 performs routing of image A, and outputs it as image B via an output port 3804. Device A (3302) outputs, as image C, the input image B. Image C is input into the NMC 202 again via the input port 3802. The SCC 305 outputs, to a TP output port 3803, image C from the input port 3802.

As a result of this, image C output from the TP output port 3803 is output to the TP 221, and image C is displayed in the preview area 3606 (S374). In other words, the image based on the looped-back video signals is output to the preview area 3606 of the TP 221.

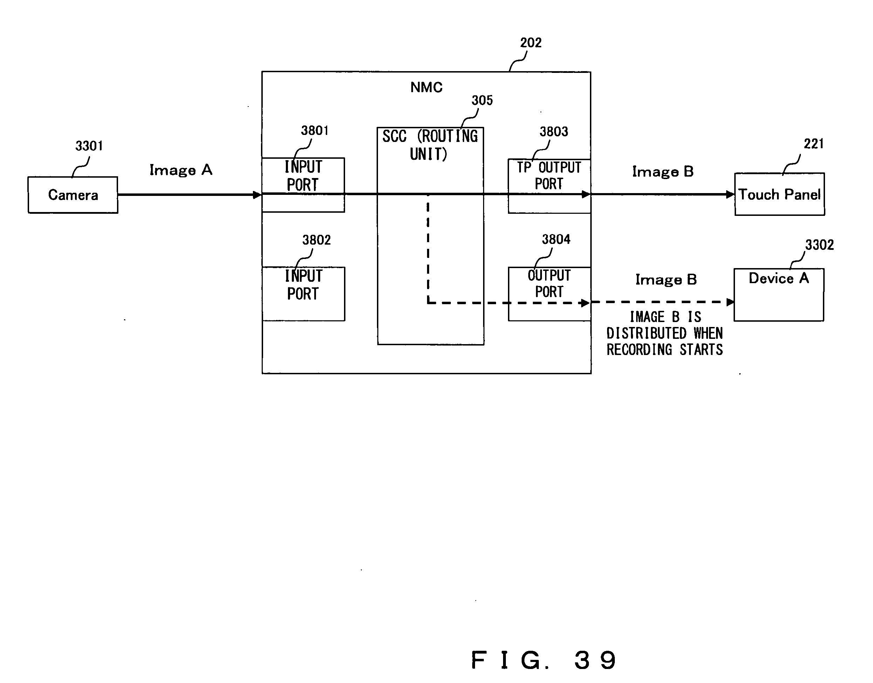

When looping back is not set in S372, the control unit 902 outputs the input video signals via the TP output port 3803 (S375). This will be explained in Fig. 39.

Fig. 39 is a flowchart for the video signals when looping back is not set according to the present embodiment. The image signals (image A) output from the camera head of the endoscope (camera 3301) is input into the NMC 202 via the input port 3801. In accordance with instructions from the control unit 902, the SCC 305 performs routing of image A, and outputs it as image B via the TP output port 3803.

As a result of this, image B output from a TP output port 113 is output to the TP 221, and image B is displayed in the preview area 3606 (S376). When a recording operation starts with the "record" button being pressed, the SCC 305 distributes image B to device A. Also, it is possible to display the image before the recording operation starts.

Accordingly, when looping back is set, the image displayed in the preview area of the TP 221 is an image that has passed through device A. Accordingly, the image signal is unerringly output to device A. Accordingly, when a recording operation starts, the image can be unerringly recorded on device A.

According to the present embodiment, the medical support control system includes the recording device (for example, device A), the medical support control device (NMC 202), and the display manipulation device (TP 221). The recording device records image signals output from medical devices. The medical support control device receives image signals output from at least one of the medical devices. The medical support control device outputs the input image signals to the recording device and records them, and causes the image signals recorded in the recording device to be input again into the medical control devices. The medical control devices can synthesize the images based on the image signals with a prescribed graphical user interface (GUI).

The display manipulation device can display the GUI containing the synthesized image. Also, the display manipulation device can input information to the GUI.

In this configuration, the medical support control device can create a prescribed GUI, and can cause the GUI to be displayed as the manipulator window (for example, the operation window 3600) on the basis of the input of the setting information for the recording device in the administrator setting window (setting window 3500) displayed on the display manipulation device. The manipulator window includes device selectionmeans (such as the tabs 3600) for selecting one of the medical devices, image selection means (such as the input video selection unit 3603) for selecting one of the image signals to be output from the medical device, and display means (such as the preview area 3606) for displaying the image corresponding to the selected image signals.

Conventionally, an image to be stored is displayed on a preview window in the TP 221 and confirmed; thereafter the previewed video signals are output to the recording device in order to be recorded. However, when a cable between the NMC and the recording device is disconnected, it is not possible to record the video signals on the recording device even when the preview window is displayed.

However, according to the present embodiment, on the basis of the loop-back information set in advance, the video signals input into the NMC 202 are output to the TP 221 via the recording device. Thereby, when an image is displayed on the preview area 3606 of the TP 221, there is no disorder such as disconnection or the like of the cable connecting the NMC and the storage device; accordingly, when a recording operation is performed in that case, the image can be unerringly recorded on the recording device.

Also, in the conventional techniques, confirmation of a plurality of windows is required in order to confirm functions of a plurality of devices. Accordingly, operations have been very complicated. However, according to the present embodiment, it is possible to display settings regarding the output on one manipulation window in a concentrated manner. As a result of this, operators only have to arbitrarily select devices from among input sources and output devices set in advance, which simplifies the operations.

Additionally, the scope of the present invention is not limited to any of the above embodiments, and various other configurations and embodiments are allowed without departing from the spirit of the present invention.

As described above, it is possible to provide the medical support control device for controlling medical devices and non-medical devices.

## Claims

1. A medical support control system that can control a medical device (100), comprising:
a plurality of video interface cards that are detachable from the medical support control system (100) and that are used for the medical support control system (100) for converting, when a video signal is input from an external environment, the video signal input from an external environment into a common signal and vice versa, said common signal being different from any video signals input into and output from the plurality of video interface cards (304) and said common signal being used commonly in the medical support control system (100);
a switching control card (305) for selecting, from among the video interface cards (304), a video interface card (304) as an output destination in accordance with an instruction given from an external environment; and
a plurality of video processing cards (307) that are detachable from the medical support control system (100) and that are used for the medical support control system (100) for processing the common signal into a video signal appropriate to the selected video interface card (304) on the basis of a video signal expressed by the common signal.

2. The medical support control system (100) according to claim 1, wherein:
the common signal is a serial signal.

3. The medical support control system (100) according to claim 1, wherein:
all of the respective video interface cards (304) have common connectors (406).

4. The medical support control system (100) according to claim 1, wherein:
the switching control card (305) determines an output destination for the common signal by detecting a position of a video interface card (304) on the basis of identification information of the video interface card (304) set as an output destination from an external environment, using video interface related information, obtained from the respective video interface cards (304), containing identification information used for identifying the respective video interface cards and position information specifying a position of the video interface card (304).
